(19) Europäisches Patentamt
European Patent Office
Office européen des brevets

(11) EP 4 180 036 A1

(12) EUROPEAN PATENT APPLICATION
published in accordance with Art. 153(4) EPC

(43) Date of publication:
**17.05.2023 Bulletin 2023/20**

(21) Application number: **20956398.0**

(22) Date of filing: **29.09.2020**

(51) International Patent Classification (IPC):
***A61K 31/40*** (2006.01) ***A61K 31/216*** (2006.01)
***A61P 3/06*** (2006.01)

(52) Cooperative Patent Classification (CPC):
**A61K 31/216; A61K 31/40; A61P 3/06**

(86) International application number:
**PCT/MX2020/050034**

(87) International publication number:
**WO 2022/071787 (07.04.2022 Gazette 2022/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Laboratorios Silanes, S.A. de C.V.**
**Mexico, 11000 (MX)**

(72) Inventors:
- **OLLERVIDES RUBIO, Paola Yazmín**
  **México, 11000 (MX)**
- **GONZÁLEZ CANUDAS, Jorge Alejandro**
  **México, 11000 (MX)**
- **ESPINOZA LEÓN, Sixto Serafín**
  **México, 11000 (MX)**
- **FARFÁN SALAZAR, Claudia Delfina**
  **México, 11000 (MX)**

(74) Representative: **Araujo, Daniel**
**Ingenias Creaciones**
**Signos e Invenciones S.L.P.**
**Avda Diagonal 514 Planta 1, Puerta 4**
**ES-08006 Barcelona (ES)**

# (54) PHARMACEUTICAL COMBINATIONS OF STATINS AND FIBRATES FOR THE TREATMENT AND PREVENTION OF HYPERLIPIDAEMIA AND CARDIOVASCULAR DISEASES

(57) This invention combines in a single dosage form the active principles, atorvastatin and fenofibrate, which represents a set of important technological challenges due to the physicochemical properties and the difference in dosage to ensure obtaining a stable product in the treatment of hyperlipidemia and the prevention of cardiovascular diseases.

EP 4 180 036 A1

## Description

### TECHNICAL FIELD

This invention relates to pharmaceutical compositions for combination in the field of the treatment of hyperlipidemias and cardiovascular diseases.

### BACKGROUND

Patent US5273995 with equivalent in Mexico MX 178535 of WARNER LAMBERT COMPANY LLC describes the active ingredient atorvastatin, a pharmaceutical composition containing it, and its use to manufacture a pharmaceutical product to alleviate hypercholesterolemia; however, their validity ended in 2011 and 2010, respectively. Patent MX 214428, proprietary of the same company describes the crystalline form I of atorvastatin hydrate having X-ray powder diffraction and containing at least one of the following 2Θ values determined by using CuKα radiation: 11.9 or 22.0. Crystalline forms II and IV of atorvastatin are also described.

Other crystalline forms of atorvastatin, and compositions containing them, have been described in patents MX 210754, MX 238174, MX 263277, 238946, MX 260435, and in patent application MX/a/2013/015272 submitted by DSM SINOCHEM PHARMACEUTICALS NETHERLANDS B.V.

Independently, French patent FR 2157853 of FOURNIER GMBH LAB describes the active ingredient fenofibrate. Consequently, patent MX 252667, co-owned by ALKERMES PHARMA IRELAND LIMITED, and ABBOTT LABORATORIES, IRELAND, LIMITED describes in its main claim a stable fenofibrate composition for oral administration, comprising fenofibrate particles having an effective average particle size of less than approximately 2000 nm.

However, there is no description of the combination of these active ingredients -atorvastatin and fenofibrate- in the same pharmaceutical form that addresses the stability of the same given the different physicochemical properties and the difference in which they are dosed to ensure that a therapeutic or preventive result is obtained. For example, atorvastatin presents a sensitivity to humidity that jeopardizes its stability and consequently the composition or pharmaceutical form containing it. For example, pharmaceutical compositions that combine these active ingredients in separate entities in a single solid dosage form, in multilayer tablets, bilayer tablets, or capsules, in solution, or sachets containing the active ingredients in separate granules or pellets have been proposed.

Mexican patent MX 333517, of ABBOTT LABORATORIES, IRELAND, LIMITED describes the use of a compound selected from fenofibrate, fenofibric acid, and a salt of fenofibric acid for the preparation of a pharmaceutical product for the treatment of obstructive sleep apnea or obstructive sleep apnea syndrome, which may be associated with a known hydroxymethylglutaryl coenzyme A reductase (HMG-CoA) inhibitor or statin, including but not limited to atorvastatin.

Mexican patent MX 270015 of SARL GALENIX INNOVATIONS describes a process for the manufacture of a pharmaceutical composition containing the active ingredient fenofibrate or one of its derivatives, possibly in the form of an association of fenofibrate or its derivative with a second active ingredient, in the form of tablets, wherein the second active ingredient is selected from metformin, cobalamin, folic acid, betaine, n-acetylcysteine, vitamin E, and an HGM-CoA inhibitor. The claimed process comprises exclusively granulation or compaction stages which are conducted by dry process.

Also patent MX 261110 of ABBOTT GMBH & CO. KG describes a formulation comprising fenofibric acid, or a physiologically acceptable salt or derivative thereof and optionally other active substances (which in one embodiment includes atorvastatin), as well as a binder component comprising at least one enteric binder and physiologically acceptable excipients, wherein the enteric binder is an enteric polymer selected from hydroxypropylmethyl cellulose phthalate, hydroxypropylmethyl cellulose acetate succinate, carboxymethylethyl cellulose, cellulose acetate phthalate, cellulose acetate trimelittate, and sodium carboxymethyl cellulose.

Separately, document WO2014195900A2 refers to a combination of a statin and some other lipid-lowering agent, citing fenofibrate as an example. In this case, a wet granulation process is employed to form tablets, capsules, or pellets.

Documents WO2006037344A1 and CN102188419 cite a solid dispersion process, where fenofibrate is melted together with other excipients and the liquid mixture is spread over a diluent. Atorvastatin coated granules are additionally made.

Document US20110165239A1 mentions the manufacture of a multilayer tablet by physically separating one part of the tablet containing fenofibrate from another part containing atorvastatin.

Mexican patent application MX/a/2013/006332 describes a cosolvent system consisting of poloxamer, polyethylene glycol and diethylene glycol monoethyl ether used to achieve the dissolution of fenofibrate. The patent refers to the fact that they cannot achieve the dissolution of the drug without the co-solvent system in the proper proportions. The co-solvent is in a proportion of 5 to 15%. It also features binders among which may be HPMC, PVP, HPC, carboxymethyl cellulose, methocel, methacrylates, and mixtures thereof. They are found in a weight proportion between 2 and 8%; antistatic agents are selected from silicon dioxide and talc, and mixtures thereof; stabilizers calcium carbonate, silicified crystalline cellulose, calcium phosphate, and mixtures thereof in a proportion of 15 to 60%. Likewise, the tablet may or may not be coated. In this application MX/a/2013/006332 the fenofibrate is incorporated via an aqueous dispersion composed of co-solvents, the surfactant, humectant, and binder. The dispersion is aspersed to the mixture of diluents and disintegrants. It is dried and sieved.

Atorvastatin, lubricant, stabilizer, antistatic agent, and compressibility vehicle are added. In conclusion, the process is a wet granulation of fenofibrate. Dissolution is ensured by the co-solvent system. However, as mentioned, atorvastatin is sensitive to moisture, and in the wet granulation process there is residual moisture that jeopardizes the stability of atorvastatin and therefore, of the tablet.

The application PA/a/2006/003813 in general describes the combination of atorvastatin-fenofibrate in concentrations of 10-80 mg and 140-170 mg respectively, as well as the pharmaceutical form in tablets. Such compositions are manufactured without any need to add water or an aqueous medium and where at least 80% of the active substances (in this case fibrates and statin) are present in the composition in dissolved form, which ensures an appropriate bioavailability of both active ingredients during oral administration.

However, in this document PA/a/2006/003813 the particulate material comprises as active substances one or more fibrates and one or more statins, wherein at least 80% of the total amount of the active substances is dissolved in a vehicle selected from the group consisting of a hydrophobic, a hydrophilic, and a water miscible vehicle. The active ingredients are dissolved in the vehicle, the active substances are present in the form of a solid solution in the particulate composition. The presence of a solid solution can be proved by a DSC test. However, some crystallization of the active substances from the solid solutions can be expected during storage. The vehicle has oil characteristics (sorption material), with a maximum melting point of 25°C; and hydrophobic or hydrophilic vehicle with melting point from 0 to 250°C, but an oil sorption material is needed to be added. They meet with melt binders or solid solvents. Examples are described including Polyethylene glycol. A mixture of PEG: poloxamer, specifically PEG 6000, is described. As a conditioner, vehicles with low melting points are preferred. In the liquid vehicle, the drugs are dissolved, and the resulting solution is sprayed onto a solid diluent. A method option to melt the vehicle and dissolve the fenofibrate is mentioned, the solution is sprayed onto a solid carrier capable of adsorbing the solution. Another method option to dissolve a solid solution of fenofibrate in a vehicle is mentioned. As part of their manufacturing process, the need to dissolve the drugs in cosolvents is indicated.

Separately, application MX/a/2010/014200 describes a formulation of fenofibrate with improved oral bioavailability, simplicity of design and manufacture, and absence of food effect, wherein the fenofibrate alone or together with statin is dissolved in a lipophilic and hydrophilic surfactant until a clear solution is obtained. It may include a pH stabilizer, antioxidants, preservatives, color, flavor, buffer solution, and viscosity agents. It features polysorbate 80 from 20 to 80%; poloxamer 10 to 20%, and the dosage form is liquid contained in a capsule. The formulation may also contain atorvastatin from 5 to 80 mg. Additionally, the invention relates to the manufacturing processes of the formulation and to the dosage forms comprising the formulation such as a soft gelatin capsule.

None of the prior state of the art documents makes the most of the physicochemical, rheological, and particle size properties while they allow combining atorvastatin and fenofibrate in a single solid, stable, and immediate release dosage form.

As mentioned, for example, atorvastatin exhibits sensitivity to moisture, and in typical granulation processes, such as wet granulation, residual moisture that jeopardizes the stability of atorvastatin, and therefore of any pharmaceutical form manufactured with such methodology containing atorvastatin, is present. In this invention, the proposed composition and its method of manufacture overcomes these major disadvantages-the use of cosolvents is not needed to achieve the dissolution effects of the active principles.

Thus, this invention proposes the combination in a single dosage form of the active principles, atorvastatin and fenofibrate, with doses of 20 $\pm$ 0.7 mg and 160-200 mg respectively solving a set of important technological challenges due to the physicochemical properties and the difference in doses to guarantee the achievement of a stable product in the treatment of hyperlipidemias and the prevention of cardiovascular diseases.

## SUMMARY OF THE INVENTION

This invention relates to solid, stable, immediate-release pharmaceutical compositions, as well as a method for manufacturing the same, comprising atorvastatin and fenofibrate, or a pharmaceutically acceptable salt thereof, for the treatment of hyperlipidemias and the prevention of cardiovascular diseases. The method makes it possible to obtain a pharmaceutical composition easy to administer in a single dose. The method includes a granulation stage activated by heating with fenofibrate, an atorvastatin incorporation stage, a compression stage wherein the process is conducted without the incorporation of water avoiding conditions that affect the stability of atorvastatin.

In one embodiment, the fenofibrate is preferably micronized. The particle size distribution of the micronized

fenofibrate is less than 30 μm (100%), preferably equal to or less than 0.95 μm (d10), preferably equal to or less than 5.35 μm (d50), and more preferably equal to or less than 11.28 μm (d90).

In another embodiment of the invention, atorvastatin is preferably in its calcium trihydrate form.

In another embodiment of the invention, the hyperlipidemias and cardiovascular diseases are selected from: hyperlipidemia, hyperlipoproteinemia, hypercholesterolemia, hypertension, angina pectoris, heart attacks, aneurysms, regulation of the blood concentration of LDL, HDL, and triglycerides.

One embodiment of the invention comprises a pharmaceutical composition comprising (a) atorvastatin, or an equivalent amount of a salt thereof, in a pharmaceutically acceptable amount in the range between 20 ± 0.7 mg and (b) micronized fenofibrate, or its micronized salt, in a pharmaceutically acceptable amount in the range between 160 mg and 200 mg, and (c) a pharmaceutically acceptable amount of pharmaceutically acceptable excipients and/or pharmaceutical vehicles. Pharmaceutically acceptable excipients and/or vehicles comprise binders, diluents, disintegrants, pH modulators, surfactants, lubricants, solvents, and coatings. In an additional embodiment of the invention, the coating functions as a moisture barrier.

In another embodiment of the invention, the composition is in the form of tablets, caplets, granules, pills. Preferably, in the form of a biconvex tablet and/or a coated tablet.

In another embodiment of the invention, the dissolution profiles of this pharmaceutical combination represent a comparison between both dosages to demonstrate bioexemption. Since this is a study of exemption to the bioequivalence test, the drug that requires exemption to this test is called the test drug. The reference drug is the one used in the comparative bioavailability study between the drugs when administered in the same formulation with respect to the reference drugs administered together and separately.

In another modality of the invention, a pharmacokinetic simulation was performed in order to predict the behavior of atorvastatin and fenofibric acid administered concomitantly, after following a dosage schedule of one tablet every 24 hours for 2 weeks, which allows demonstrating that the concentrations reached for both drugs are effective, safe, and comparable to those reported in the literature.

## BRIEF DESCRIPTION OF THE FIGURES

**Figure 1.** Average particle size distribution analysis for fenofibrate.

**Figure 2.** Average percentages dissolved according to the sampling times for the reference drug, percentage of dissolved atorvastatin.

**Figure 3.** Average percentages dissolved according to the sampling times of the test drug, percentage of dissolved atorvastatin.

**Figure 4.** Average percentages dissolved according to the sampling times of the reference drug, percentage of dissolved fenofibrate.

**Figure 5.** Average percentages dissolved according to the sampling times of the test drug, percentage of dissolved fenofibrate.

**Figure 6.** Average percentages of atorvastatin (reference and test drug) dissolved at the sampling times performed.

**Figure 7.** Average percentages of fenofibrate (reference and test drug) dissolved at the sampling times performed.

**Figure 8.** Bicompartmental model of plasma concentrations of atorvastatin.

**Figure 9.** Compartmental pharmacokinetic modeling for atorvastatin.

**Figure 10.** Simulation of atorvastatin plasma concentrations at steady state after administration of one tablet with atorvastatin / fenofibrate (20 mg / 200 mg) every 24 hours for 14 days.

**Figure 11.** Single-compartment model of plasma concentrations of fenofibric acid.

**Figure 12.** Compartmental pharmacokinetic modeling for atorvastatin.

**Figure 13.** Simulation of plasma concentrations of fenofibric acid at steady state, after administration of one tablet with atorvastatin / fenofibrate (20 mg / 200 mg) every 24 hours for 14 days.

**Figure 14.** Comparison of the fenofibrate dissolution profile between a commercial fenofibrate tablet (reference), a composition described in the prior state of the art (patent application MX/a/2013/006332), against the fenofibrate and atorvastatin composition of this invention (test) was performed.

**Figure 15.** Comparison of the atorvastatin dissolution profile between a commercial atorvastatin tablet (reference), a composition described in the prior state of the art (patent application MX/a/2013/006332), against the fenofibrate and atorvastatin composition of this invention (test) was also performed.

## DETAILED DESCRIPTION OF THE INVENTION

### *Definitions*

Pharmaceutically Acceptable Salt: The term Pharmaceutically Acceptable Salt of a given compound refers to salts that retain the biological efficacy and properties of the given compound, and are not biologically, or otherwise, undesirable (P. Heinrich Stahl and Camille G. Wermuth (Eds.) Pharmaceutical Salts Properties, Selection, and Use (International Union of Pure and Applied Chemistry), Wiley-VCH; 2a Revised Edition (May 16, 2011)). Pharmaceutically acceptable base addition salts can be prepared from inorganic or organic bases. Salts derived from inorganic bases include, by way of example only, sodium, potassium, lithium, ammonium, calcium, and magnesium salts. Salts derived from organic bases include, but are not limited to, salts of primary, secondary, and tertiary amines. Specific examples of suitable amines include isopropylamine, trimethylamine, diethylamine, tri (isopropyl) amine, tri (n-propyl) amine, ethanolamine, 2-dimethylaminoethanol, tromethamine, lysine, arginine, histidine, caffeine, procaine, hydrabamine, choline, betaine, ethylenediamine, glucosamine, N-alkylglucamines, theobromine, purines, piperazine, piperidine, morpholine, N-ethylpiperidine, and the like.

Pharmaceutically acceptable acid addition salts can be prepared from inorganic or organic acids. Salts derived from inorganic acids include hydrochloric acid, hydrobromic acid, sulfuric acid, nitric acid, phosphoric acid, and the like. Salts derived from organic acids include acetic acid, propionic acid, glycolic acid, pyruvic acid, oxalic acid, malic acid, malonic acid, succinic acid, maleic acid, maleic acid, fumaric acid, tartaric acid, citric acid, benzoic acid, cinnamic acid, mandelic acid, methanesulfonic acid, ethanesulfonic acid, p-toluensulfonic acid, salicylic acid, and the like.

Excipient: is the ingredient that is part of the present pharmaceutical composition. Excipients can be diluents, disintegrants, lubricants, coating, absorbents, among others.

Stability. It is the ability of a pharmaceutical product to retain its chemical, physical, microbiological, and biopharmaceutical properties within specified limits throughout its shelf life.

This invention relates to solid, stable, immediate-release, synergistically effective, dissolution-maintaining, and bioavailable pharmaceutical compositions of a statin and a fibrate, which are administered in a single pharmaceutical form orally as a therapeutic agent for the treatment of hyperlipidemias and the prevention of cardiovascular diseases that are selected from: hyperlipidemia, hyperlipoproteinemia, hypercholesterolemia, hypertension, angina pectoris, heart attacks, aneurysms, regulation of the blood concentration of LDL, HDL, and triglycerides.

This invention also relates to the manufacture of said compositions of a statin and a fibrate for oral administration. Particularly, the statin is selected from atorvastatin and the fibrate is selected from fenofibrate. The method makes it possible to obtain a pharmaceutical composition easy to administer in a single dose.

The combination of atorvastatin and fenofibrate represents a set of important technological challenges due to the physicochemical properties of the drugs, the proper selection of excipients and manufacturing conditions, as well as the difference in dosage to ensure obtaining a stable product in the development of pharmaceutical compositions, a very important role in relation to the release of the drug, the rate of absorption, and amount absorbed in the body.

The composition of this invention comprises (a) atorvastatin in a pharmaceutically acceptable amount in the range between 20 $\pm$ 0.7 mg and (b) fenofibrate in a pharmaceutically acceptable amount in the range between 160 mg and 200 mg, and (c) a pharmaceutically acceptable amount of pharmaceutically acceptable excipients and/or pharmaceutical vehicles. The pharmaceutical composition can be used for the manufacture of a drug product useful in the treatment of hyperlipidemias and the prevention of cardiovascular diseases.

In one embodiment, the composition of this invention comprises atorvastatin or an equivalent amount of a salt thereof, preferably in its calcium trihydrate form, wherein 21.65 mg of atorvastatin calcium trihydrate is equivalent to 20 mg of atorvastatin.

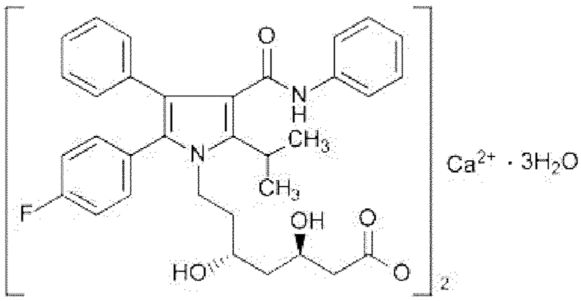

In a preferred embodiment, the pharmaceutically acceptable amount of the active principle atorvastatin is adjusted according to titration.

In another embodiment, the composition of the present invention comprises fenofibrate preferably in a micronized form.

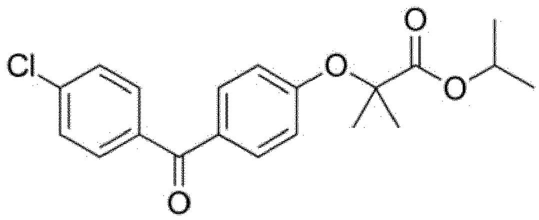

The person skilled in the art may realize that various methodologies for micronization of particles exist in the prior state of the art, such as Remington Pharmaceuticals. Gennaro AR, editor. 20th Edition. Tome I. Buenos Aires: Editorial Médica Panamericana; Lachman L, Lieberman HA, Kanig JL. The theory and practice of industrial pharmacy 3 ed. Philadelphia: Lea-Febiger, 1986:333; and Pharmacopoeias: USP, British Pharmacopoeia. It will be evident for a technician in the field to follow or combine the existing pharmaceutical documents to solve and perform micronization, reduction, and particle size distribution processes, where multiple variations are possible in the realization of this process without departing from the spirit and scope of the same to ensure the proper functioning of the product and meet the required quality characteristics.

The determination of the particle size distribution by laser beam diffraction for micronized fenofibrate was carried out using a DPS module in a particle size analyzer.

The particle size distribution of the micronized fenofibrate is less than 30 μm (100%), preferably the particle size distribution of the micronized fenofibrate is equal to or less than 0.95 μm (d10), preferably equal to or less than 5.35 μm (d50), and more preferably equal to or less than 11.28 μm (d90) (Figure 1).

The pharmaceutical composition of the present invention comprises pharmaceutically acceptable excipients and/or vehicles comprising, but not limited to, binders, diluents, disintegrants, pH modulators, surfactants, lubricants, solvents, and coatings.

Examples of pharmaceutically acceptable binders are various grades of polyethylene glycol, glyceryl behenate, microcrystalline wax, stearoyl polyoxyglycerides. In one embodiment, the binder is preferably poloxamer 188 in a pharmaceutically acceptable amount in the range of 5 to 10%.

Examples of pharmaceutically acceptable diluents, whose function known in the prior state of the art include adjusting and maintaining constant tablet weight, include, but are not limited to, cellulose derivatives such as PH102 microcrystalline cellulose, phosphate derivatives such as dibasic calcium phosphate, starch derivatives such as pregelatinized starch and corn starch, as well as mannitol, xylitol, maltitol, lactitol, sorbitol, sucrose, or combinations thereof. In one embodiment, the diluent is preferably lactose monohydrate in a pharmaceutically acceptable amount in the range of 5 to 90%. In a further embodiment, the diluent is also preferably magnesium aluminum silicate in a pharmaceutically acceptable amount in the range of 5 to 90%.

Pharmaceutically acceptable disintegrants include, but are not limited to, croscarmellose, cellulose derivatives such as hydroxypropyl cellulose, carboxymethyl cellulose, microcrystalline cellulose, povidone derivatives such as crospovidone, starch derivatives such as pregelatinized starch, sodium starch glycolate, and corn starch. In one embodiment, the disintegrant is preferably starch sodium glycolate, which is selected in this invention for its high capacity to rapidly uptake water and increase its volume, in a pharmaceutically acceptable amount in the range of 2 to 8%.

Examples of pH modulators for providing formulation stability comprise alkali and alkaline earth metal salts, such as calcium phosphate, sodium hydroxide, sodium carbonate, sodium bicarbonate, calcium hydroxide. In one embodiment, the pH modulator is preferably magnesium oxide in a pharmaceutically acceptable amount in the range of 0.5 to 5%.

Pharmaceutically acceptable surfactants may be such as poloxamers, polyoxyethylene castor oil derivatives, benzalkonium chloride, benzethonium chloride, polyoxyethylene alkyl ethers, and polyoxyethylene sorbitan fatty acid esters. In one embodiment, the surfactant is preferably sodium lauryl sulfate in a pharmaceutically acceptable amount in the range of 1 to 2.5%.

Examples of pharmaceutically acceptable lubricants include, but are not limited to, magnesium stearate, zinc stearate, calcium stearate, stearic acid, monostearate, stearyl fumarate; talc, and sulfated derivatives such as magnesium lauryl sulfate. In one embodiment, the lubricant is preferably magnesium stearate in a pharmaceutically acceptable amount in the range of 0.25 to 5%.

On the other hand, the composition of this invention may contain coatings, which may be in an enunciative, and non-limiting manner selected from cellulose derivatives such as hydroxypropylmethyl cellulose, hydroxypropyl cellulose, carboxymethyl celluloses; polyvinyl derivatives such as polyvinyl alcohol; polyethylene glycol, povidones in all grades K and their derivatives, as well as moisture barrier coatings. In one embodiment, the moisture barrier coating is preferably Opadry, such as Opadry AMB II blue in a pharmaceutically acceptable amount in the range of 0.5 to 6%.

The composition of this invention also comprises solvent, such as and preferably water in a pharmaceutically acceptable or enough amount (q.s.).

This invention also describes an optimized method that permits the combined manufacture of the active principles atorvastatin and fenofibrate in a single solid, stable, immediate-release pharmaceutical form. Atorvastatin is preferably in its calcium trihydrate form, wherein 21.65 mg of atorvastatin calcium trihydrate is equivalent to 20 mg of atorvastatin.

The solid pharmaceutical composition is selected from the group comprising a tablet, monolayer tablet, granules, caplet, or pills. Preferably, in the form of a tablet and/or a coated tablet. The tablets with biconvex shape allow to perform the coating process efficiently, achieving a stable product with good physical and chemical characteristics.

The most preferred pharmaceutical form of the invention is "tablets" due to its dosage accuracy. It is also the pharmaceutical form with the best acceptance due to its easy administration, making it possible to dose a high concentration of drugs, unlike capsules. In the case of tablets, it is possible to reduce the volume of the powders and thus facilitate their handling and administration.

This invention also refers both to the pharmaceutical composition and to the manufacturing method for obtaining it. The method makes it possible to obtain a pharmaceutical composition easy to administer in a single dose.

The production process of the composition in tablet form lies in the selection of the unitary operations, the order, and the execution time to control the different physicochemical properties of the drugs. The technological challenge lies in the combination of two drugs with very low water solubility and poor flow. In the case of fenofibrate, a preferably micronized material is used, so it is necessary to carry out a granulation process to manufacture tablets, considering the high concentration of the active ingredient. Atorvastatin also presents important stability problems, being sensitive to humidity, acid pH and high temperatures.

In a melt granulation process, the binder solution of a standard wet granulation process is replaced by a fusible binder. This binder can be added molten, but it can also be added in its solid state when the technology employed allows it.

However, in this invention the process is not a melt granulation since the melting temperature of the binder is not reached. Heat-activated granulation is a process in which heating is used to trigger granule formation (i.e., agglomeration) in the presence of a binder with a manageable glass transition temperature without the need to reach a melting temperature. The glass transition temperature (Tg) is the temperature above which a reversible transition occurs in which the non-crystalline regions of the polymer change from a glassy state (rigid and brittle) to a state called viscoelastic, with a significant loss of rigidity. It is associated with the onset of long-range movement of the polymer chain due to temperature factors. By raising the temperature above the glass transition temperature, a viscoelastic behavior of the polymer is achieved, i.e., the polymer in its solid state is made to exhibit mechanical characteristics intermediate between its liquid and solid-state characteristics.

The objective of granulation activated by heating is to achieve densification of the powder mixture to improve flow and compaction, as well as drug dissolution.

Poloxamer 188 has a melting point between 52°C and 57°C, reporting a glass transition temperature around 50°C. The process is operated at a product temperature of 47°C, at which temperature the polymer achieves adhesion of smaller particles (fine powders) to form larger particles (granules or agglomerates).

Temperature control is a critical point since, as mentioned, it is only required to densify the powder mixture. Managing the product temperature at the melting temperature of the polymer implies a greater adhesion of the particles, i.e., the granule formed will be larger and more rigid, which are crucial factors in the dissolution of the drug.

Among the characteristics of fenofibrate, the negative electrostatic charges are found, which dissipate with the incorporation of magnesium oxide, so that handling in subsequent stages ensures better handling and total incorporation of the drug.

Fenofibrate shows low solubility, light sensitivity, elastic deformation, and moderate adhesiveness. Magnesium oxide, as already mentioned, dissipates the charges of fenofibrate, but also achieves greater particle slippage and decreases the adhesiveness of the drug.

Magnesium aluminum silicate is employed as a diluent, but not as a moisture adsorbent since it improves the rheological properties of fenofibrate by decreasing the electrostatic charges without affecting the flow characteristics together with magnesium oxide.

Thus, in this invention, to conduct a process of granulation activated by heating with fenofibrate and to incorporate atorvastatin to carry out the manufacture without the incorporation of water and avoiding conditions that could affect the stability of this drug is determined. The manufacture was carried out seeking to keep the process free of water, due to the sensitivity of atorvastatin; a granulation process is required since the characteristics of the fenofibrate powder do not enable a process by direct compression.

In another embodiment of the invention, the dissolution rate of the active principles was determined, and the dissolution profiles of the present pharmaceutical combination represent a comparison between both dosages to demonstrate exemption.

Due to the innovation made in the process and formulation of the drug, it was possible to obtain a product with proven stability through studies in accordance with current regulations in which the quality attributes were met during the evaluation period. Stability tests are a means to compare different formulations, packaging materials, or manufacturing processes in short-term experiments. As soon as the final formulation and manufacturing process are established, the manufacturer conducts a series of stability tests that will predict the stability of the product or drug, in this case, and determine its shelf life and storage conditions.

The initial and accelerated results for the determination of the stability of the compositions of this invention for the manufacture of a drug product useful in the treatment of hyperlipidemias and the prevention of cardiovascular diseases in the condition of 40°C/75%RH, 25°C/60%RH and 30°C/75%RH are shown below in Examples 10-15 (Tables 20-61), wherein the pharmaceutical compositions for the drug product are presented in dosages of 20/160 mg and 20/200 mg for atorvastatin / fenofibrate respectively:
The technological development involved a series of evaluations defining the formulation and process as described below. The person skilled in the art will find that multiple variations and modalities are possible in the realization of this invention without departing from the spirit and scope of this invention to ensure the proper functioning of the product and to meet the required quality characteristics.

## Examples

### Example 1. Particle size distribution of fenofibrate.

Determination of particle size distribution by laser beam diffraction was carried out for micronized fenofibrate by placing 0.5 g of the raw material in a sample holder and using a DPS module in the particle size analyzer to obtain an obscuration of 4-7%. The determination is made in duplicate.

The particle size distribution of the micronized fenofibrate is less than 30 μm (100%), preferably equal to or less than 0.95 μm (d10), preferably equal to or less than 5.35 μm (d50), and more preferably equal to or less than 11.28 μm (d90) (Figure 1).

### Example 2. Manufacturing process to produce tablets.

The manufacturing process for the preparation of tablets comprising the pharmaceutical composition of the present invention comprises:

- a stage of granulation activated by heating with the fenofibrate, preferably micronized fenofibrate.
- a stage of incorporation of the atorvastatin.
- a compression stage.

wherein the process is carried out without the incorporation of water avoiding conditions affecting the stability of the atorvastatin.

In this sense, the various stages include:

1. 70% magnesium oxide is added to the micronized fenofibrate and manually mixed to eliminate powder static.

2. The above mixture is sieved by mesh (0.64 mm or 0.025 in.), along with poloxamer 188, 20% lactose monohydrate DCL-11, and 80% magnesium aluminum silicate.

3. The powder is loaded into a granulating equipment with heating system and brought to a temperature of 47°C with constant motion.

4. When the temperature is reached, the heating is stopped and the granulate obtained is left to cool to 25-30°C.

5. The granules obtained in step 3 are subjected to a particle size reduction operation (1.27 mm or 0.050 in.).

6. Atorvastatin and 20% magnesium aluminum silicate are added to a diffusion mixer, mixing for 3 minutes.

7. Sodium starch glycolate, 30% magnesium oxide, and 80% lactose monohydrate DCL-11 are sieved through a mesh (1.27 mm or 0.050 in.).

8. The sieved product from step 5 and the powder from step 7 are added to the mixer from step 6 and mixed for 5 min.

9. Sodium lauryl sulfate and magnesium stearate are sieved through a mesh (1.27 mm or 0.050 in.).

10. The powder of step 9 is added to the mixer from step 8 and mixed for 3 min.

11. The final powder mixture is compressed into 600 ± 30 mg nuclei, with a hardness of 6.0-13.0 Kp and disintegration time of less than 9 min.

12. The cores are coated with 3.23% Opadry AMB II in weight gain.

**Example 3. Pharmaceutical composition of atorvastatin in combination with fenofibrate.**

Table 1. Pharmaceutical composition of atorvastatin in combination with fenofibrate.

| Ingredients | Quantity (mg) | Observations |
|---|---|---|
| Atorvastatin (or atorvastatin calcium trihydrate) | 20 ± 0.7 (21.65) | Active ingredient |
| Fenofibrate | 160 - 200 | Micronized active ingredient |
| Poloxamer (e.g., poloxamer 188) | 45 - 55 | Binder |
| Lactose monohydrate | 199 - 241 | Diluent |
| Aluminum magnesium silicate | 45 - 55 | Diluent |
| Sodium starch glycolate | 45 - 52 | Disintegrant |
| Magnesium oxide | 8 - 12 | Modular pH |
| Sodium lauryl sulfate | 10 - 20 | Surfactant |
| Magnesium stearate | 1 - 10 | Lubricant |
| Opadry AMB II | 15 - 25 | Moisture barrier coating polymer |
| Water | q.s. | Solvent |

Enough water (q.s.) for the formulation comprises the water of the coating and is up to 0.08 mg/tab without affecting the manufacturing process and stability of the pharmaceutical composition of the invention.

**Example 4. Preferred pharmaceutical composition of atorvastatin in combination with fenofibrate.**

Table 2. Preferred pharmaceutical composition of atorvastatin in combination with fenofibrate.

| Ingredients | Quantity (mg) | Observations |
|---|---|---|
| Atorvastati n | 20.68 | Active ingredient |

(continued)

| Ingredients | Quantity (mg) | Observations |
|---|---|---|
| Fenofibrate | 160 | Micronized active ingredient |
| Poloxamer (e.g., poloxamer 188) | 50 | Binder, e.g. |
| Lactose monohydrate | 239 | Diluent |
| Aluminum magnesium silicate | 50 | Diluent |
| Sodium starch glycolate | 48 | Disintegrant |
| Magnesium oxide | 10.35 | Modular pH |
| Sodium lauryl sulfate | 15 | Surfactant |
| Magnesium stearate | 6 | Lubricant |
| Opadry AMB II | 20 | Moisture barrier coating polymer |
| Water | q.s. | Solvent |

Enough water (q.s.) for the formulation comprises the water of the coating and is up to 0.08 mg/tab without affecting the manufacturing process and stability of the pharmaceutical composition of the invention.

**Example 5. Preferred pharmaceutical composition of atorvastatin in combination with fenofibrate.**

Table 3. Preferred pharmaceutical composition of atorvastatin in combination with fenofibrate.

| Ingredients | Quantity (mg) | Observations |
|---|---|---|
| Atorvastatin | 20.68 | Active ingredient |
| Fenofibrate | 200 | Micronized active ingredient |
| Poloxamer (e.g., poloxamer 188) | 50 | Binder |
| Lactose monohydrate | 199 | Diluent |
| Aluminum magnesium silicate | 50 | Diluent |
| Sodium starch glycolate | 48 | Disintegrant |
| Magnesium oxide | 10.35 | Modular pH |
| Sodium lauryl sulfate | 15 | Surfactant |
| Magnesium stearate | 6 | Lubricant |
| Opadry AMB II | 20 | Moisture barrier coating polymer |
| Water | q.s. | Solvent |

Statins and fenofibric acid derivatives (fibrates) are widely used for the treatment of hypercholesterolemia and hypertriglyceridemia, respectively, and when administered together, they have shown efficacy in the treatment of mixed hyperlipidemia.

Once through a manufacturing process that incorporates granulation activated by heating and compression without the intervention of water ensuring stable conditions, the active ingredients, atorvastatin and fenofibrate, are combined in a dose of 20/200 mg and 20/160 mg respectively, it is essential to verify that a single solid, stable, and immediate release dosage form has been obtained. For this purpose, dissolution and pharmacokinetic tests and profiles are presented below.

**Example 6. Dissolution tests.**

The dissolution test (*in vitro* test) serves to determine the rate (amount/time) and extent (total amount) at which

a drug is released from the dosage form; in the case of the dissolution profile, it corresponds to the quantification at different times of the dissolved drug under standardized conditions. The importance of the dissolution test lies in the following:

a) It is a guide for the development of new formulations during product development: it allows evaluating the possible interference of excipients or the manufacturing process on drug release.

b) Process control and quality assurance: it helps to ensure the continuous quality of the product and its optimization after a change in manufacturing, formulation, manufacturing site, and process scale-up.

c) *In vivo* development indicator: it is an indicator of bioavailability; it allows establishing the correlation between *in vitro* parameters with bioavailability results.

Dissolution profiles were performed for atorvastatin / fenofibrate tablets 20/200 mg (reference drug) and atorvastatin / fenofibrate tablets 20/160 mg (test drug). Both drug lots (reference and test) are property of Laboratorios Silanes S.A. de C.V.

The development, execution of the test, and obtaining of results was performed following the criteria and specifications established in the Mexican Official Standard NOM-177-SSA1-2013. The comparison of the dissolution profiles was performed using the $f_2$ similarity factor method. In the case of atorvastatin, a similarity factor $f_2$ = 68.03 was found and for fenofibrate, a similarity factor $f_2$ = 59.91. As established by the Mexican Official Standard NOM-177-SSA1-2013, when a similarity factor is greater than or equal to 50, the dissolution profiles are considered similar.

For sample analysis, two dissolution methods were implemented; one to quantify atorvastatin in dissolution profile samples by HPLC based on the guidelines described in the United States Pharmacopeia USP, Ed. 41, 2018. This method was validated according to NOM-177-SSA1-2013.

An Agilent Technologies model 708-DS dissolver using 0.05 M phosphate buffer pH 6.8 as dissolution medium, and an Agilent Technologies model 1200 chromatograph were employed.

For fenofibrate, another method was implemented to quantify dissolution profile samples by HPLC as described in the United States Pharmacopeia USP, Ed. 41, 2018. This method was validated according to NOM-177-SSA1-2013. An Agilent Technologies model 708-DS dissolver using 0.05 M sodium dodecyl sulfate solution as dissolution medium, and an Agilent Technologies model 1200 chromatograph were employed.

The percentage of atorvastatin dissolved in its respective dissolution medium for both the reference drug and the test drug was greater than 80% on average within the first 15 min, and greater than 90% on average within 20 min, so these results show that the evaluated products can be accepted as equivalent. See Tables 4-5 and Figure 2- 3.

Table 4. Reference drug, dissolved atorvastatin.

| Reference drug | | | | | |
|---|---|---|---|---|---|
| Dissolved Atorvastatin % | | | | | |
| | Time (minutes) | | | | |
| | 10 | 15 | 20 | 25 | 30 |
| Average | 60.25 | 80.37 | 90.56 | 96.23 | 98.05 |
| CV% | 8.54 | 8.21 | 7.74 | 5.74 | 3.66 |

Table 5. Test drug, dissolved atorvastatin.

| Test drug | | | | | |
|---|---|---|---|---|---|
| Dissolved Atorvastatin % | | | | | |
| | Time (minutes) | | | | |
| | 10 | 15 | 20 | 25 | 30 |
| Average | 65.65 | 86.31 | 93.22 | 95.55 | 95.24 |
| CV% | 16.04 | 9.13 | 5.14 | 3.32 | 2.36 |

The percentage of fenofibrate dissolved in its respective dissolution medium for both the reference drug and the test drug was greater than 40% on average within the first 20 min, and greater than 80% on average within 20 min, so these results show that the evaluated products can be accepted as equivalent. See Tables 6-7 and Figures 4-5.

Table 6. Reference drug, dissolved fenofibrate.

| Reference drug | | | | | |
|---|---|---|---|---|---|
| Dissolved Fenofibrate % | | | | | |
| | Time (minutes) | | | | |
| | 15 | 20 | 30 | 45 | 60 |
| Average | 39.75 | 44.97 | 63.86 | 78.12 | 87.42 |
| CV% | 19.81 | 9.98 | 7.93 | 6.38 | 7.27 |

Table 7. Test drug, dissolved fenofibrate.

| Test drug | | | | | |
|---|---|---|---|---|---|
| Dissolved Fenofibrate % | | | | | |
| | Time (minutes) | | | | |
| | 15 | 20 | 30 | 45 | 60 |
| Average | 43.14 | 54.07 | 73.82 | 79.45 | 86.73 |
| CV% | 10.78 | 8.62 | 6.81 | 7.45 | 5.38 |

**Example 7. Dissolution profiles.**

The average dissolved percentages of each drug (reference and test drug) at the sampling times performed and the calculation of the similarity factor for atorvastatin (Tables 8-9) and fenofibrate (Tables 10-11) are shown in the form of a comparative table and graph. See Figures 6 and 7.

Table 8. Comparison % of dissolved atorvastatin for the reference drug and the test drug.

| Dissolved Atorvastatin % | | |
|---|---|---|
| Time (min) | Reference drug | Test drug |
| 0 | 0.00 | 0.0 |
| 10 | 60.25 | 65.65 |
| 15 | 80.37 | 86.31 |
| 20 | 90.56 | 93.22 |
| 25 | 96.23 | 95.55 |
| 30 | 98.05 | 95.24 |

Table 9. Calculation of the atorvastatin similarity factor.

| Time (min) | Reference % Dissolved | Test % Dissolved | Difference | Sum of squares | n | $f_2$ |
|---|---|---|---|---|---|---|
| 10 | 60.25 | 65.65 | -5.39 | 71.97 | 4 | 68.03 |
| 15 | 80.37 | 86.31 | -5.94 | Similar dissolution profiles | | |
| 20 | 90.56 | 93.22 | -2.66 | | | |
| 25 | 96.23 | 95.55 | 0.68 | | | |

Table 10. Comparison % of dissolved fenofibrate for the reference drug and the test drug.

| Dissolved Fenofibrate % | | |
|---|---|---|
| Time (min) | Reference drug | Test drug |
| 0 | 0.00 | 0.00 |
| 15 | 39.75 | 43.14 |
| 20 | 44.97 | 54.07 |
| 30 | 63.86 | 73.82 |
| 45 | 78.12 | 79.45 |
| 60 | 87.42 | 86.73 |

Table 11. Calculation of the fenofibrate similarity factor.

| Time (min) | Reference % Dissolved | Test % Dissolved | Difference | Sum of squares | n | $f_2$ |
|---|---|---|---|---|---|---|
| 15 | 39.75 | 43.14 | -3.39 | 195.72 | 5 | 59.91 |
| 20 | 44.97 | 54.07 | -9.10 | Similar dissolution profiles | | |
| 30 | 63.86 | 73.82 | -9.95 | | | |
| 45 | 78.12 | 79.45 | -1.33 | | | |
| 60 | 87.42 | 86.73 | 0.69 | | | |

Under the conditions evaluated for atorvastatin, it can be appreciated that both drugs have similar dissolution profiles. The comparison was performed considering the average values of the first four sampling times, from the first sampling time until maximum one sampling time after the reference drug reached 85% of the dissolved drug. The similarity factor was f2 = 68.03. The Mexican Official Standard NOM-177-SSA1-2013 states that if the similarity factor is $\geq$ 50, the dissolution profiles are considered similar.

Under the conditions evaluated for fenofibrate, it can be appreciated that the dissolution profiles present a tendency to be similar. The comparison of the dissolution profiles was performed by calculating the similarity factor $f_2$. In this case, the results of the five sampling times were considered. The similarity factor was $f_2$ = 59.91, and therefore the profiles are considered similar as established by the Mexican Official Standard NOM-177-SSA1-2013.

**Example 8. Pharmacokinetic simulation of atorvastatin and fenofibrate at steady state.**

Based on international criteria, the performance of a clinical pharmacokinetic interaction study with single-dose administration is acceptable to characterize the association between atorvastatin and fenofibrate, being the in-silico extrapolation predictive or representative of the bioavailability that would be obtained, following a multiple-dose schedule until reaching a steady state (FDA, 2020).

The pharmacokinetic simulation was performed with the aim of predicting the behavior of atorvastatin and fenofibric acid administered concomitantly, after following a dosing schedule of one tablet every 24 hours for 2 weeks, which demonstrates that the concentrations achieved for both drugs are effective, safe, and comparable to what is reported in the literature, from 3.17-30 μm/mL for fenofibric acid (Back, 2018; Moffat, 2011) and 2.5-50.1 ng/mL for atorvastatin (Chou, 2013).

The plasma concentration data with respect to time for atorvastatin and fenofibric acid (active metabolite of fenofibrate), on which the simulation to steady state is based, corresponds to those obtained in the study performed in healthy volunteers, in fasting condition, to demonstrate the no pharmacokinetic interaction between atorvastatin 20 mg and fenofibrate 200 mg, when administered in fixed dose (pharmaceutical product property of Laboratorios Silanes, S.A. de C.V.) versus the individual reference drugs indicated by the Federal Commission for Protection against Sanitary Risks (COFEPRIS).

The product of interest corresponds to the one manufactured by Laboratorios Silanes with the following description:

Test drug (treatment B)

Generic name: atorvastatin / fenofibrate
Pharmaceutical form: tablets
Formula: Each tablet contains: atorvastatin 20 mg / fenofibrate 200 mg
Dosage administered: 20 mg atorvastatin / 200 mg fenofibrate (one tablet)
Batch: 19A081G1

***Pharmacokinetic simulation at steady* state *(atorvastatin)***

The mean plasma concentration profiles and descriptive statistics of the main pharmacokinetic parameters of atorvastatin after administration of a single oral dose of the fixed combination with atorvastatin 20 mg / fenofibrate 200 mg are shown below (Table 12 and Table 13):

Table 12. Descriptive statistics for plasma concentrations of atorvastatin with respect to time for treatment B (test drug in fixed combination).

| Time (h) | N | Mean (ng/mL) | SD (ng/mL) | SE (ng/mL) | Min (ng/mL) | Median (ng/mL) | Max (ng/mL) | CV% |
|---|---|---|---|---|---|---|---|---|
| 0.00 | 38 | 0.000 | 0.000 | 0.000 | 0.00 | 0.00 | 0.00 | - |
| 0.25 | 38 | 1.594 | 2.258 | 0.366 | 0.00 | 0.63 | 8.11 | 141.7 |
| 0.50 | 38 | 7.510 | 7.880 | 1.278 | 0.00 | 4.68 | 36.34 | 104.9 |
| 0.75 | 38 | 10.835 | 7.939 | 1.288 | 0.00 | 9.00 | 29.85 | 73.3 |
| 1.00 | 38 | 8.731 | 4.532 | 0.735 | 0.34 | 7.66 | 21.42 | 51.9 |
| 1.33 | 38 | 7.159 | 3.989 | 0.647 | 1.35 | 5.91 | 18.47 | 55.7 |
| 1.67 | 38 | 5.368 | 2.341 | 0.380 | 1.93 | 4.96 | 11.87 | 43.6 |
| 2.00 | 38 | 4.488 | 2.157 | 0.350 | 1.50 | 3.64 | 10.32 | 48.0 |
| 2.50 | 38 | 3.799 | 2.206 | 0.358 | 1.37 | 3.29 | 11.22 | 58.1 |
| 3.00 | 38 | 3.424 | 2.219 | 0.360 | 0.90 | 2.79 | 9.92 | 64.8 |
| 3.50 | 38 | 2.694 | 1.508 | 0.245 | 0.78 | 2.26 | 7.47 | 56.0 |
| 4.00 | 38 | 2.523 | 1.360 | 0.221 | 0.75 | 2.06 | 5.81 | 53.9 |
| 4.50 | 38 | 3.184 | 1.738 | 0.282 | 0.73 | 2.86 | 6.86 | 54.6 |
| 5.00 | 38 | 2.900 | 1.513 | 0.245 | 0.63 | 2.65 | 6.70 | 52.2 |
| 5.50 | 38 | 2.956 | 1.651 | 0.268 | 0.51 | 2.66 | 8.55 | 55.9 |
| 6.00 | 38 | 2.781 | 1.529 | 0.248 | 0.52 | 2.39 | 7.13 | 55.0 |
| 8.00 | 38 | 2.175 | 1.059 | 0.172 | 0.40 | 1.91 | 4.51 | 48.7 |
| 12.00 | 38 | 1.253 | 0.573 | 0.093 | 0.37 | 1.10 | 2.68 | 45.7 |
| 24.00 | 38 | 0.334 | 0.189 | 0.031 | 0.00 | 0.33 | 0.65 | 56.6 |
| 36.00 | 38 | 0.258 | 0.219 | 0.036 | 0.00 | 0.30 | 0.74 | 85.1 |
| 48.00 | 36 | 0.028 | 0.083 | 0.014 | 0.00 | 0.00 | 0.35 | 294.9 |
| 72.00 | 36 | 0.000 | 0.000 | 0.000 | 0.00 | 0.00 | 0.00 | - |
| 96.00 | 37 | 0.000 | 0.000 | 0.000 | 0.00 | 0.00 | 0.00 | - |

Table 13. Descriptive statistics of pharmacokinetic parameters of atorvastatin for treatment B (fixed-combination test drug).

| Variable | Treatment | N | Mean Geom. | Mean | SD | SE | Min | Me | Max | C.V. % |
|---|---|---|---|---|---|---|---|---|---|---|
| Tmax (h) | B | 38 | 0.893 | 0.969 | 0.471 | 0.076 | 0.50 | 0.75 | 3.00 | 48.6 |
| Cmax (ng/mL) | B | 38 | 12.493 | 14.005 | 7.205 | 1.169 | 4.92 | 12.29 | 36.34 | 51.4 |
| $ABC_{0-t}$ (h*ng/mL) | B | 38 | 44.350 | 49.140 | 21.856 | 3.545 | 13.70 | 45.90 | 96.71 | 44.5 |
| $ABC_{0-inf}$ (h*ng/mL) | B | 38 | 49.415 | 53.626 | 21.695 | 3.519 | 20.08 | 51.66 | 100.90 | 40.5 |
| Ke (1/h) | B | 38 | 0.086 | 0.092 | 0.036 | 0.006 | 0.04 | 0.09 | 0.20 | 39.0 |
| $t_{1/2}$ (h) | B | 38 | 8.056 | 8.641 | 3.287 | 0.533 | 3.50 | 7.95 | 16.31 | 38.0 |

***Compartmental Pharmacokinetic Modeling for Atorvastatin***

The simulation of plasma concentrations at steady state was performed by fitting the data to the best compartmental pharmacokinetic model using the Phoenix/Winnonlin version 8.2 software. In this sense, the plasma concentrations of atorvastatin have a better correlation with a bicompartmental model, represented schematically (Fig. 8, 9 and 10) and mathematically (Tables 14 and 15):
*Equation 1*

$$Cp(t) = Ae^{-alpha*t} + Be^{-beta*t} + Ce^{-K01t} \quad (1)$$

Table 14. Initial parameters to perform the simulation with atorvastatin.

| Parameter | Units | Estimated | Lower | Upper |
|---|---|---|---|---|
| V1_F | mg/(ng/mL) | 1.029 | 0 | 10.29 |
| K01 | 1/h | 3.623 | 0 | 36.23 |
| K10 | 1/h | 0.3858 | 0 | 3.858 |
| K12 | 1/h | 1.264 | 0 | 12.64 |
| K21 | 1/h | 0.5311 | 0 | 5.311 |
| Tlag | h | 0.2471 | 0 | 2.471 |

Table 15. Simulation parameters of plasma concentrations of atorvastatin at steady state

| Parameter | Units | Estimated | VIF | Sqrt[VIF]_P_% |
|---|---|---|---|---|
| AUC | h*ng/mL | 50.379331 | 112.90935 | 21.091747 |
| K01_HL | h | 0.19131857 | 0.047415023 | 113.81535 |
| K10_HL | h | 1.796649 | 3.0507873 | 97.217115 |
| Alpha | 1/h | 2.0825099 | 6.1125471 | 118.72004 |
| Beta | 1/h | 0.098390111 | 0.0014574227 | 38.800857 |
| Alpha_HL | h | 0.3328422 | 0.15597262 | 118.65493 |
| Beta_HL | h | 7.0448866 | 7.4395057 | 38.716673 |
| A | ng/mL | 35.742318 | 23047.522 | 424.74611 |
| B | ng/mL | 4.3571332 | 1.1598659 | 24.717415 |
| CL_F | mL/h | 396988.2 | 7025.0344 | 21.112839 |
| V2_F | mL | 2448985.1 | 737427.95 | 35.064984 |

(continued)

| Parameter | Units | Estimated | VIF | Sqrt[VIF]_P_% |
|---|---|---|---|---|
| CLD2_F | mL/h | 1300656 | 650001.62 | 61.986165 |
| Tmax | h | 0.67257989 | 0.0054556714 | 10.981969 |
| Cmax | ng/mL | 10.330577 | 0.47093023 | 6.6428388 |

Based on the above results, the accumulation factor was calculated with the following Equation 2:

$$R = \frac{Cmax_{ee}}{Cmax_1} = \frac{1}{1 - e^{-beta * \tau}} \qquad (2)$$

Where R is the accumulation factor, beta is the elimination constant in the bicompartmental model, and T is the dosing interval (24 hours for the simulation). The value of R = 1.10, which means that there is no significant accumulation (10%) of atorvastatin at steady state when administered once daily.

***Simulated steady-state pharmacokinetics (fenofibric acid)***

The mean plasma concentration profiles and descriptive statistics of the main pharmacokinetic parameters of fenofibric acid (main active metabolite of fenofibrate) after administration of a single oral dose of the fixed combination with atorvastatin 20 mg / fenofibrate 200 mg are shown below (Table 16 and Table 17).

Table 16. Descriptive statistics for plasma concentrations of fenofibric acid with respect to time for treatment B (fixed-combination test drug).

| Time (h) | N | Mean (μg/mL) | SD (μg/mL) | SE (μg/mL) | Min (μg/mL) | Median (μg/mL) | Max (μg/mL) | CV% |
|---|---|---|---|---|---|---|---|---|
| 0.00 | 38 | 0.000 | 0.000 | 0.000 | 0.00 | 0.00 | 0.00 | - |
| 0.25 | 38 | 0.000 | 0.000 | 0.000 | 0.00 | 0.00 | 0.00 | - |
| 0.50 | 38 | 0.105 | 0.129 | 0.021 | 0.00 | 0.05 | 0.43 | 123.4 |
| 0.75 | 38 | 0.347 | 0.339 | 0.055 | 0.00 | 0.26 | 1.51 | 97.6 |
| 1.00 | 38 | 0.591 | 0.502 | 0.081 | 0.00 | 0.51 | 2.24 | 85.0 |
| 1.33 | 38 | 0.879 | 0.622 | 0.101 | 0.00 | 0.78 | 2.94 | 70.8 |
| 1.67 | 38 | 1.056 | 0.626 | 0.101 | 0.10 | 1.03 | 3.18 | 59.3 |
| 2.00 | 38 | 1.237 | 0.658 | 0.107 | 0.26 | 1.17 | 3.39 | 53.2 |
| 2.50 | 38 | 1.490 | 0.778 | 0.126 | 0.37 | 1.43 | 3.83 | 52.2 |
| 3.00 | 38 | 1.656 | 0.794 | 0.129 | 0.39 | 1.57 | 4.01 | 47.9 |
| 3.50 | 38 | 1.902 | 0.975 | 0.158 | 0.38 | 1.88 | 4.74 | 51.3 |
| 4.00 | 38 | 1.932 | 0.919 | 0.149 | 0.39 | 1.87 | 4.95 | 47.5 |
| 4.50 | 38 | 2.004 | 0.926 | 0.150 | 0.41 | 1.85 | 4.85 | 46.2 |
| 5.00 | 38 | 1.851 | 0.879 | 0.143 | 0.37 | 1.68 | 4.80 | 47.5 |
| 5.50 | 38 | 1.881 | 0.890 | 0.144 | 0.40 | 1.64 | 4.86 | 47.3 |
| 6.00 | 38 | 1.934 | 0.921 | 0.149 | 0.43 | 1.67 | 4.93 | 47.6 |
| 8.00 | 38 | 1.874 | 0.841 | 0.136 | 0.55 | 1.70 | 4.70 | 44.9 |
| 12.00 | 38 | 1.571 | 0.607 | 0.099 | 0.60 | 1.46 | 3.29 | 38.7 |
| 24.00 | 38 | 1.337 | 0.466 | 0.076 | 0.63 | 1.22 | 2.27 | 34.9 |
| 36.00 | 38 | 1.298 | 0.455 | 0.074 | 0.51 | 1.20 | 2.51 | 35.1 |
| 48.00 | 36 | 1.060 | 0.409 | 0.068 | 0.36 | 1.01 | 2.04 | 38.6 |

(continued)

| Time (h) | N | Mean (μg/mL) | SD (μg/mL) | SE (μg/mL) | Min (μg/mL) | Median (μg/mL) | Max (μg/mL) | CV% |
|---|---|---|---|---|---|---|---|---|
| 72.00 | 37 | 0.566 | 0.356 | 0.059 | 0.00 | 0.47 | 1.61 | 62.9 |
| 96.00 | 37 | 0.266 | 0.187 | 0.031 | 0.00 | 0.28 | 0.66 | 70.3 |

Table 17. Descriptive statistics of pharmacokinetic parameters of fenofibric acid by treatment.

| Variable | Tre atm ent | N | Mean Geom. | Mean | SD | SE | Min | Me | Max | C.V.% |
|---|---|---|---|---|---|---|---|---|---|---|
| Tmax (h) | B | 38 | 6.494 | 9.886 | 11.198 | 1.816 | 2.50 | 4.50 | 35.67 | 113.3 |
| Cmax (ng/mL) | B | 38 | 2.017 | 2.194 | 0.944 | 0.153 | 0.95 | 2.02 | 4.95 | 43.0 |
| $ABC_{0-t}$ (h*ng/mL) | B | 38 | 88.188 | 93.674 | 32.811 | 5.323 | 40.28 | 88.97 | 165.88 | 35.0 |
| $ABC_{0-inf}$ (h*ng/mL) | B | 38 | 100.87 1 | 107.430 | 38.006 | 6.165 | 43.72 | 101.89 | 186.55 | 35.4 |
| Ke (1/h) | B | 38 | 0.027 | 0.028 | 0.006 | 0.001 | 0.01 | 0.03 | 0.04 | 23.1 |
| $t_{1/2}$ (h) | B | 38 | 25.653 | 26.426 | 6.884 | 1.117 | 16.43 | 25.32 | 51.24 | 26.0 |

***Compartmental Pharmacokinetic Modeling for Fenofibric Acid***

The simulation of plasma concentrations at steady state was performed by fitting the data to the best compartmental pharmacokinetic model using the Phoenix/Winnonlin version 8.2 software. In this sense, the plasma concentrations of fenofibric acid correlate best with a single compartmental model, represented schematically (Fig. 11, 12 and 13) and mathematically (Tables 18 and 19):
*Equation 3*

$$Cp(t) = \frac{D * K01}{V\,(K01 - K10)} * (e^{-K10*t} - e^{-K01*t}) \qquad (3)$$

Table 18. Initial parameters for starting the simulation with fenofibric acid.

| Parameter | Unit | Estimated | Lower | Upper |
|---|---|---|---|---|
| V_F | mg/(ng/mL) | 90.93 | 0 | 909.3 |
| K01 | 1/h | 0.5759 | 0 | 5.759 |
| K10 | 1/h | 0.02051 | 0 | 0.2051 |
| Tlag | h | 0.4326 | 0 | 4.326 |

Table 19. Simulation parameters of plasma concentrations of fenofibric acid at steady state

| Parameter | Units | Estimated | VIF | Sqrt[VIF]_P_% |
|---|---|---|---|---|
| AUC | h*ug/mL | 107.24008 | 985.51533 | 29.273493 |
| K01_HL | h | 1.2035895 | 1.4446528 | 99.862714 |
| K10_HL | h | 33.795572 | 302.21492 | 51.439667 |
| CL_F | mL/h | 1864.9743 | 298.6503 | 29.302767 |
| Tmax | h | 6.4374281 | 12.38848 | 54.675986 |
| Cmax | ug/mL | 1.9446235 | 0.13957299 | 19.211673 |

Based on the above results, the accumulation factor was calculated with the following Equation 4:

$$R = \frac{Cmax_{ee}}{Cmax_1} = \frac{1}{1 - e^{-K10 * \tau}} \quad (4)$$

Where R is the accumulation factor, K10 is the elimination constant in the monocompartmental model and T is the dosing interval (24 hours for the simulation).

The value of R indicates a 2.57-fold accumulation in the plasma concentrations of fenofibric acid at steady state when administered once a day.

In conclusion, the projection to steady state complements the results obtained in the interaction study where both drugs were administered in single doses, and which in essence provides sufficient pharmacokinetic information considering the following reasons:

✓ The pharmacokinetic parameters of atorvastatin are not significantly different when comparing single and multiple dosing for two weeks, in patients on hemodialysis (Lins, 2003) or 90 days in patients with daily intake of grapefruit juice (Reddy, 2011).

✓ Atorvastatin does not show accumulation at steady state in the dosing schedule once a day.

✓ The extent of absorption and concentrations of atorvastatin increase in a dose-proportional manner (Lipitor, 2020).

✓ Atorvastatin and fenofibrate (fenofibric acid) do not have time-dependent pharmacokinetics (e.g., auto-inhibition or auto-induction).

✓ The processes responsible for the elimination of atorvastatin from the systemic circulation is CYP3A4, P-gp and OATP2(OATP1B1), but fenofibrate and fenofibric acid are not metabolized via cytochrome P450, do not significantly inhibit P-gp-mediated transport, and do not have a potential to cause a clinically significant drug-drug interaction by inhibition of OATP1B1 (Wong, 2006, Yamazaki, 2005).

**Example 9. Comparison of the dilution profile of fenofibrate and atorvastatin.**

Comparison of the fenofibrate dissolution profile (Fig. 14) between a commercial fenofibrate tablet (reference), a composition described in the prior state of the art (patent application MX/a/2013/006332), against the fenofibrate and atorvastatin composition of this invention (test) was performed.

Comparison of the atorvastatin dissolution profile (Fig. 15) between a commercial atorvastatin tablet (reference), a composition described in the prior state of the art (patent application MX/a/2013/006332), against the fenofibrate and atorvastatin composition of this invention (test) was also performed.

As observed, the dissolution profile of the fenofibrate-atorvastatin composition of this invention is better than that of the individual active ingredients, as well as that of the combination described in the prior state of the art.

**Example 10. Stability of pharmaceutical compositions of atorvastatin and fenofibrate 20/160 mg tablets.**

**Accelerated Stability Analytical Results (40°C ± 2°C / 75% RH ± 5% RH)**

Table 20. Product description results

| Specification | | Initial | 1 month | 3 months | 6 months |
|---|---|---|---|---|---|
| Blue coated biconvex tablet, smooth on both sides | Lot 1 | Blue coated biconvex tablet, smooth on both sides | Blue coated biconvex tablet, smooth on both sides | Blue coated biconvex tablet, smooth on both sides | Blue coated biconvex tablet, smooth on both sides |
| | Lot 2 | Blue coated biconvex tablet, smooth on both sides | Blue coated biconvex tablet, smooth on both sides | Blue coated biconvex tablet, smooth on both sides | Blue coated biconvex tablet, smooth on both sides |
| | Lot 3 | Blue coated biconvex tablet, smooth on both sides | Blue coated biconvex tablet, smooth on both sides | Blue coated biconvex tablet, smooth on both sides | Blue coated biconvex tablet, smooth on both sides |

Table 21. Atorvastatin content results

| Specification | | Initial | 1 month | 3 months | 6 months |
|---|---|---|---|---|---|
| 90.0% to 110.0% | **Lot 1** | 103.4% | 107.1% | 104.1% | 102.9% |
| | **Lot 2** | 99.4% | 107.7% | 102.1% | 104.3% |
| | **Lot 3** | 99 2% | 106.9% | 104.9% | 105.1% |

Table 22. Fenofibrate content results

| Specification | | Initial | 1 month | 3 months | 6 months |
|---|---|---|---|---|---|
| 90.0% to 110.0% | **Lot 1** | 99.5% | 100.2% | 96.0% | 96.2% |
| | **Lot 2** | 95.0% | 100.6% | 95.3% | 95.5% |
| | **Lot 3** | 97.0% | 101.6% | 97.1% | 99.3% |

Table 23. Organic impurities of atorvastatin results

| Specification | | Initial | 1 month | 3 months | 6 months |
|---|---|---|---|---|---|
| Atorvastatin Pyrrolidone Analog<br>No more than 0.90% | **Lot 1** | 0.16% | 0.14% | 0.17% | 0.17% |
| | **Lot 2** | 0.16% | 0.13% | 0.19% | 0.19% |
| | **Lot 3** | 0.17% | 0.14% | 0.18% | 0.17% |
| Related Compound H<br>No more than 1.40% | **Lot 1** | 0.05% | 0.03% | 0.00% | 0.04% |
| | **Lot 2** | 0.05% | 0.00% | 0.00% | 0.03% |
| | **Lot 3** | 0.02% | 0.02% | 0.02% | 0.03% |
| Epoxy Pyrrolooxazin 6-Hydroxy Analog<br>No more than 0.90% | **Lot 1** | 0.00% | 0.00% | 0.00% | 0.39% |
| | **Lot 2** | 0.00% | 0.00% | 0.00% | 0.46% |
| | **Lot 3** | 0.02% | 0.00% | 0.00% | 0.43% |
| Epoxy Pyrrolooxazin 7-Hydroxy Analog<br>No more than 0.90% | **Lot 1** | 0.00% | 0.04% | 0.01% | 0.00% |
| | **Lot 2** | 0.00% | 0.00% | 0.01% | 0.00% |
| | **Lot 3** | 0.16% | 0.04% | 0.06% | 0.00% |

(continued)

| Specification | | Initial | 1 month | 3 months | 6 months |
|---|---|---|---|---|---|
| Epoxy Tetrahydrofuran Analog<br>No more than 1.00% | Lot 1 | 0.00% | 0.00% | 0.00% | 0.00% |
| | Lot 2 | 0.00% | 0.00% | 0.00% | 0.00% |
| | Lot 3 | 0.00% | 0.00% | 0.00% | 0.01% |
| Related Compound D when integrated with Epoxy Tetrahydrofuran Analog<br>No more than 0.75% | Lot 1 | 0.02% | 0.00% | 0.00% | 0.01% |
| | Lot 2 | 0.02% | 0.00% | 0.00% | 0.01% |
| | Lot 3 | 0.02% | 0.00% | 0.00% | 0.01% |
| Any other unspecified degradation products<br>Not more than 0.60% | Lot 1 | 0.16% | 0.20% | 0.30% | 0.17% |
| | Lot 2 | 0.15% | 0.20% | 0.30% | 0.18% |
| | Lot 3 | 0.09% | 0.20% | 0.20% | 0.19% |
| Total degradation products<br>No more than 5.00% | Lot 1 | 0.56% | 1.50% | 1.30% | 1.12% |
| | Lot 2 | 0.55% | 1.10% | 1.80% | 1.19% |
| | Lot 3 | 0.68% | 1.70% | 1.10% | 1.14% |

Table 24. Organic impurities of fenofibrate results

| Specification | | Initial | 1 month | 3 month | 6 months |
|---|---|---|---|---|---|
| Related Compound A<br>No more than 0.60% | Lot 1 | 0.00% | 0.00% | 0.00% | 0.00% |
| | Lot 2 | 0.00% | 0.00% | 0.00% | 0.00% |
| | Lot 3 | 0.00% | 0.00% | 0.00% | 0.00% |
| Related Compound B<br>No more than 0.90% | Lot 1 | 0.01% | 0.00% | 0.00% | 0.01% |
| | Lot 2 | 0.01% | 0.00% | 0.00% | 0.01% |
| | Lot 3 | 0.01% | 0.00% | 0.00% | 0.01% |
| Any unspecified impurities<br>No more than 0.60% | Lot 1 | 0.13% | 0.10% | 0.10% | 0.08% |
| | Lot 2 | 0.12% | 0.10% | 0.10% | 0.09% |
| | Lot 3 | 0.10% | 0.10% | 0.10% | 0.09% |
| Total impurities<br>No more than 1.50% | Lot 1 | 0.16% | 0.10% | 0.10% | 0.14% |
| | Lot 2 | 0.15% | 0.10% | 0.10% | 0.15% |
| | Lot 3 | 0.14% | 0.10% | 0.10% | 0.15% |

Table 25. Atorvastatin dissolution results

| Specification | | Initial | 1 month | 3 months | 6 months |
|---|---|---|---|---|---|
| Q = 75.0% in 30 minutes | Lot 1 | 104.0% | 107.4% | 104.3% | 104.2% |
| | Lot 2 | 105.8% | 107.1% | 103.2% | 104.2% |
| | Lot 3 | 105.8% | 106.2% | 105.5% | 103.1% |

Table 26. Fenofibrate dissolution results

| Specification | | Initial | 1 month | 3 months | 6 months |
|---|---|---|---|---|---|
| Q = 75.0% in 30 minutes | **Lot 1** | 86.6% | 86.6% | 88.7% | 88.6% |
| | **Lot 2** | 84.0% | 90.6% | 88.9% | 87.1% |
| | **Lot 3** | 84.9% | 86.3% | 90.3% | 86.5% |

**Example 11. Stability of pharmaceutical compositions of atorvastatin and fenofibrate 20/160 mg tablets.**

**Accelerated Stability Analytical Results (25°C ± 2°C / 60% RH ± 5% RH)**

Table 27. Product description results

| Specification | | Initial | 1 month | 3 months | 6 months |
|---|---|---|---|---|---|
| Blue coated biconvex tablet, smooth on both sides | Lot 1 | Blue coated biconvex tablet, smooth on both sides | Blue coated biconvex tablet, smooth on both sides | Blue coated biconvex tablet, smooth on both sides | Blue coated biconvex tablet, smooth on both sides |
| | Lot 2 | Blue coated biconvex tablet, smooth on both sides | Blue coated biconvex tablet, smooth on both sides | Blue coated biconvex tablet, smooth on both sides | Blue coated biconvex tablet, smooth on both sides |
| | Lot 3 | Blue coated biconvex tablet, smooth on both sides | Blue coated biconvex tablet, smooth on both sides | Blue coated biconvex tablet, smooth on both sides | Blue coated biconvex tablet, smooth on both sides |

Table 28. Atorvastatin content results

| Specification | | Initial | 3 months | 6 months |
|---|---|---|---|---|
| 90.0% to 110.0% | **Lot 1** | 103.4% | 106.1% | 109.9% |
| | **Lot 2** | 99.4% | 104.7% | 105.1% |
| | **Lot 3** | 99.2% | 105.7% | 103.9% |

Table 29. Fenofibrate content results

| Specification | | Initial | 3 months | 6 months |
|---|---|---|---|---|
| 90.0% to 110.0% | **Lot 1** | 99.5% | 95.8% | 101.6% |
| | **Lot 2** | 95.0% | 95.2% | 99.5% |
| | **Lot 3** | 97.0% | 95.8% | 95.9% |

Table 30. Organic impurities of atorvastatin results

| Specification | | Initial | 3 months | 6 months |
|---|---|---|---|---|
| Atorvastatin Pyrrolidone Analog<br><br>No than 0.90%more | **Lot 1** | 0.16% | 0.10% | 0.32 % |
| | **Lot 2** | 0.16% | 0.10% | 0.29% |
| | **Lot 3** | 0.17% | 0.12% | 0.33% |

(continued)

| Specification | | Initial | 3 months | 6 months |
|---|---|---|---|---|
| Related Compound H<br><br>No more than 1.40% | **Lot 1** | 0.05% | 0.01% | 0.03% |
| | **Lot 2** | 0.05% | 0.01% | 0.03% |
| | **Lot 3** | 0.02% | 0.01% | 0.04% |
| Epoxy Pyrrolooxazin 6-Hydroxy Analog<br><br>No more than 0.90% | **Lot 1** | 0.00% | 0.00% | 0.20% |
| | **Lot 2** | 0.00% | 0.00% | 0.21% |
| | **Lot 3** | 0.02% | 0.00% | 0.25% |
| Epoxy Pyrrolooxazin 7-Hydroxy Analog<br><br>No more than 0.90% | **Lot 1** | 0.00% | 0.00% | 0.00% |
| | **Lot 2** | 0.00% | 0.00% | 0.00% |
| | **Lot 3** | 0.16% | 0.00% | 0.00% |
| Epoxy Tetrahydrofuran Analog<br><br>No more than 1.00% | **Lot 1** | 0.00% | 0.00% | 0.00% |
| | **Lot 2** | 0.00% | 0.00% | 0.00% |
| | **Lot 3** | 0.00% | 0.00% | 0.00% |
| Related Compound D when integrated with Epoxy Tetrahydrofuran Analog<br><br>No more than 0.75% | **Lot 1** | 0.02% | 0.10% | 0.03% |
| | **Lot 2** | 0.02% | 0.10% | 0.03% |
| | **Lot 3** | 0.02% | 0.00% | 0.03% |
| Any other unspecified degradation products<br><br>Not more than 0.60% | **Lot 1** | 0.16% | 0.10% | 0.12% |
| | **Lot 2** | 0.15% | 0.10% | 0.09% |
| | **Lot 3** | 0.09% | 0.10% | 0.11% |
| Total degradation products<br><br>No more than 5.00% | **Lot 1** | 0.56% | 0.50% | 0.94% |
| | **Lot 2** | 0.55% | 0.30% | 0.91% |
| | **Lot 3** | 0.68% | 0.30% | 1.08% |

Table 31. Organic impurities of fenofibrate results

| Specification | | Initial | 3 months | 6 months |
|---|---|---|---|---|
| Related Compound A<br><br>No more than 0.60% | **Lot 1** | 0.00% | 0.00% | 0.00% |
| | **Lot 2** | 0.00% | 0.00% | 0.00% |
| | **Lot 3** | 0.00% | 0.00% | 0.00% |
| Related Compound B<br><br>No more than 0.90% | **Lot 1** | 0.01% | 0.01% | 0.01% |
| | **Lot 2** | 0.01% | 0.01% | 0.01% |
| | **Lot 3** | 0.01% | 0.01% | 0.01% |
| Any unspecified impurities<br><br>No more than 0.60% | **Lot 1** | 0.13% | 0.09% | 0.07% |
| | **Lot 2** | 0.12% | 0.09% | 0.08% |
| | **Lot 3** | 0.10% | 0.08% | 0.09% |
| Total impurities<br><br>No more than 1.50% | **Lot 1** | 0.16% | 0.11% | 0.12% |
| | **Lot 2** | 0.15% | 0.10% | 0.13% |
| | **Lot 3** | 0.14% | 0.12% | 0.14% |

Table 32. Atorvastatin dissolution results

| Specification | | Initial | 3 months | 6 months |
|---|---|---|---|---|
| Q = 75.0% in 30 minutes | **Lot 1** | 104.0% | 108.8% | 108.6% |
| | **Lot 2** | 105.8% | 107.0% | 107.9% |
| | **Lot 3** | 105.8% | 107.4% | 106.2% |

Table 33. Fenofibrate dissolution results

| Specification | | Initial | 3 months | 6 months |
|---|---|---|---|---|
| Q = 75.0% in 30 minutes | **Lot 1** | 86.6% | 83.6% | 81.4% |
| | **Lot 2** | 84.0% | 87.0% | 84.4% |
| | **Lot 3** | 84.9% | 84.6% | 81.6% |

**Example 12. Stability of pharmaceutical compositions of atorvastatin and fenofibrate 20/160 mg tablets.**

**Accelerated Stability Analytical Results (30°C ± 2°C / 75% RH ± 5% RH)**

Table 34. Product description results

| Specification | | Initial | 1 month | 3 months | 6 months |
|---|---|---|---|---|---|
| Blue coated biconvex tablet, smooth on both sides | Lot 1 | Blue coated biconvex tablet, smooth on both sides | Blue coated biconvex tablet, smooth on both sides | Blue coated biconvex tablet, smooth on both sides | Blue coated biconvex tablet, smooth on both sides |
| | Lot 2 | Blue coated biconvex tablet, smooth on both sides | Blue coated biconvex tablet, smooth on both sides | Blue coated biconvex tablet, smooth on both sides | Blue coated biconvex tablet, smooth on both sides |
| | Lot 3 | Blue coated biconvex tablet, smooth on both sides | Blue coated biconvex tablet, smooth on both sides | Blue coated biconvex tablet, smooth on both sides | Blue coated biconvex tablet, smooth on both sides |

Table 35. Atorvastatin content results

| Specification | | Initial | 3 months | 6 months |
|---|---|---|---|---|
| 90.0% to 110.0% | **Lot 1** | 103.4% | 108.1% | 109.7% |
| | **Lot 2** | 99.4% | 109.2% | 109.0% |
| | **Lot 3** | 99.2% | 104.2% | 109.2% |

Table 36. Fenofibrate content results

| Specification | | Initial | 3 months | 6 months |
|---|---|---|---|---|
| 90.0% to 110.0% | **Lot 1** | 99.5% | 97.9% | 100.1% |
| | **Lot 2** | 95.0% | 100.4% | 99.2% |
| | **Lot 3** | 97.0% | 95.4% | 99.2% |

Table 37. Organic impurities of atorvastatin results

| Specification | | Initial | 3 months | 6 months |
|---|---|---|---|---|
| Atorvastatin Pyrrolidone Analog | **Lot 1** | 0.16% | 0.10% | 0.11% |
| | **Lot 2** | 0.16% | 0.34% | 0.28% |
| No more than 0.90% | **Lot 3** | 0.17% | 0.36% | 0.04% |
| Related Compound H | **Lot 1** | 0.05% | 0.01% | 0.04% |
| | **Lot 2** | 0.05% | 0.01% | 0.04% |
| No more than 1.40% | **Lot 3** | 0.02% | 0.02% | 0.04% |
| Epoxy Pyrrolooxazin 6-Hydroxy Analog | **Lot 1** | 0.00% | 0.00% | 0.24% |
| | **Lot 2** | 0.00% | 0.00% | 0.36% |
| No more than 0.90% | **Lot 3** | 0.02% | 0.00% | 0.27% |
| Epoxy Pyrrolooxazin 7-Hydroxy Analog | **Lot 1** | 0.00% | 0.00% | 0.00% |
| | **Lot 2** | 0.00% | 0.00% | 0.00% |
| No more than 0.90% | **Lot 3** | 0.16% | 0.00% | 0.00% |
| Epoxy Tetrahydrofuran Analog | **Lot 1** | 0.00% | 0.00% | 0.00% |
| | **Lot 2** | 0.00% | 0.00% | 0.00% |
| No more than 1.00% | **Lot 3** | 0.00% | 0.00% | 0.00% |
| Related Compound D when integrated with Epoxy Tetrahydrofuran Analog | **Lot 1** | 0.02% | 0.00% | 0.02% |
| | **Lot 2** | 0.02% | 0.10% | 0.04% |
| No more than 0.75% | **Lot 3** | 0.02% | 0.10% | 0.04% |
| Any other unspecified degradation products | **Lot 1** | 0.16% | 0.20% | 0.06% |
| | **Lot 2** | 0.15% | 0.20% | 0.09% |
| Not more than 0.60% | **Lot 3** | 0.09% | 0.30% | 0.11% |
| Total degradation products | **Lot 1** | 0.56% | 0.80% | 0.66% |
| | **Lot 2** | 0.55% | 0.80% | 1.06% |
| No more than 5.00% | **Lot 3** | 0.68% | 0.80% | 0.74% |

Table 38. Organic impurities of fenofibrate results

| Specification | | Initial | 3 months | 6 months |
|---|---|---|---|---|
| Related Compound A | **Lot 1** | 0.00% | 0.00% | 0.00% |
| | **Lot 2** | 0.00% | 0.00% | 0.00% |
| No more than 0.60% | **Lot 3** | 0.00% | 0.00% | 0.00% |

(continued)

| Specification | | Initial | 3 months | 6 months |
|---|---|---|---|---|
| Related Compound B<br><br>No more than 0.90% | **Lot 1** | 0.01% | 0.00% | 0.01% |
| | **Lot 2** | 0.01% | 0.00% | 0.01% |
| | **Lot 3** | 0.01% | 0.00% | 0.01% |
| Any unspecified impurities<br><br>No more than 0.60% | **Lot 1** | 0.13% | 0.10% | 0.10% |
| | **Lot 2** | 0.12% | 0.10% | 0.09% |
| | **Lot 3** | 0.10% | 0.10% | 0.08% |
| Total impurities<br><br>No more than 1.50% | **Lot 1** | 0.16% | 0.10% | 0.15% |
| | **Lot 2** | 0.15% | 0.10% | 0.14% |
| | **Lot 3** | 0.14% | 0.10% | 0.13% |

Table 39. Atorvastatin dissolution results

| Specification | | Initial | 3 months | 6 months |
|---|---|---|---|---|
| Q = 75.0% in 30 minutes | **Lot 1** | 104.0% | 107.9% | 106.9% |
| | **Lot 2** | 105.8% | 108.3% | 107.1% |
| | **Lot 3** | 105.8% | 106.5% | 106.9% |

Table 40. Fenofibrate dissolution results

| Specification | | Initial | 3 months | 6 months |
|---|---|---|---|---|
| Q = 75.0% in 30 minutes | **Lot 1** | 86.6% | 81.5% | 83.2% |
| | **Lot 2** | 84.0% | 84.7% | 86.7% |
| | **Lot 3** | 84.9% | 81.8% | 84.1% |

**Example 13. Stability of pharmaceutical compositions of atorvastatin and fenofibrate 20/200 mg tablets.**

**Accelerated Stability Analytical Results (40°C ± 2°C / 75% RH ± 5% RH)**

Table 41. Product description results

| Specification | | Initial | 1 month | 3 months | 6 months |
|---|---|---|---|---|---|
| White coated biconvex tablet, smooth on both sides | Lot 1 | White coated biconvex tablet, smooth on both sides | White coated biconvex tablet, smooth on both sides | White coated biconvex tablet, smooth on both sides | White coated biconvex tablet, smooth on both sides |
| | Lot 2 | White coated biconvex tablet, smooth on both sides | White coated biconvex tablet, smooth on both sides | White coated biconvex tablet, smooth on both sides | White coated biconvex tablet, smooth on both sides |
| | Lot 3 | White coated biconvex tablet, smooth on both sides | White coated biconvex tablet, smooth on both sides | White coated biconvex tablet, smooth on both sides | White coated biconvex tablet, smooth on both sides |

Table 42. Atorvastatin content results

| Specification | | Initial | 1 month | 3 months | 6 months |
|---|---|---|---|---|---|
| 90.0% to 110.0% | **Lot 1** | 100.3% | 102.8% | 102.9% | 97.0% |
| | **Lot 2** | 99.8% | 105.1% | 99.6% | 101.9% |
| | **Lot 3** | 106.3% | 106.1% | 97.0% | 99.7% |

Table 43. Fenofibrate content results

| Specification | | Initial | 1 month | 3 months | 6 months |
|---|---|---|---|---|---|
| 90.0% to 110.0% | **Lot 1** | 99.8% | 100.0% | 98.4% | 95.5% |
| | **Lot 2** | 99.9% | 99.9% | 98.5% | 97.9% |
| | **Lot 3** | 101.4% | 99.4% | 95.6% | 98.5% |

Table 44. Organic impurities of atorvastatin results

| Specification | | Initial | 1 month | 3 months | 6 months |
|---|---|---|---|---|---|
| Atorvastatin Pyrrolidone Analog<br>No more than 0.90% | **Lot 1** | 0.13% | 0.18% | 0.34% | 0.44% |
| | **Lot 2** | 0.13% | 0.15% | 0.37% | 0.80% |
| | **Lot 3** | 0.13% | 0.21% | 0.35% | 0.80% |
| Related Compound H<br>No more than 1.40% | **Lot 1** | 0.06% | 0.04% | 0.04% | 0.05% |
| | **Lot 2** | 0.06% | 0.05% | 0.05% | 0.04% |
| | **Lot 3** | 0.06% | 0.05% | 0.04% | 0.04% |
| Epoxy Pyrrolooxazin 6-Hydroxy Analog<br>No more than 0.90% | **Lot 1** | 0.00% | 0.33% | 0.49% | 0.53% |
| | **Lot 2** | 0.00% | 0.31% | 0.50% | 0.56% |
| | **Lot 3** | 0.00% | 0.32% | 0.49% | 0.56% |
| Epoxy Pyrrolooxazin 7-Hydroxy Analog<br>No more than 0.90% | **Lot 1** | 0.04% | 0.05% | 0.04% | 0.07% |
| | **Lot 2** | 0.00% | 0.05% | 0.07% | 0.11% |
| | **Lot 3** | 0.00% | 0.04% | 0.07% | 0.11% |
| Epoxy Tetrahydrofuran Analog<br>No more than 1.00% | **Lot 1** | 0.00% | 0.01% | 0.01% | 0.02% |
| | **Lot 2** | 0.00% | 0.01% | 0.01% | 0.01% |
| | **Lot 3** | 0.00% | 0.01% | 0.01% | 0.01% |
| Related Compound D when integrated with Epoxy Tetrahydrofuran Analog<br>No more than 0.75% | **Lot 1** | 0.02% | 0.04% | 0.06% | 0.07% |
| | **Lot 2** | 0.02% | 0.04% | 0.08% | 0.13% |
| | **Lot 3** | 0.02% | 0.04% | 0.06% | 0.13% |
| Any other unspecified degradation products<br>Not more than 0.60% | **Lot 1** | 0.13% | 0.10% | 0.15% | 0.14% |
| | **Lot 2** | 0.14% | 0.08% | 0.14% | 0.14% |
| | **Lot 3** | 0.15% | 0.11% | 0.15% | 0.13% |
| Total degradation products<br>No more than 5.00% | **Lot 1** | 0.56% | 1.16% | 1.73% | 1.97% |
| | **Lot 2** | 0.51% | 1.10% | 1.85% | 2.74% |
| | **Lot 3** | 0.51% | 1.20% | 1.76% | 2.65% |

Table 45. Organic impurities of fenofibrate results

| **Specification** | | **Initial** | **1 month 3 month** | | **6 months** |
|---|---|---|---|---|---|
| Related Compound A<br>No more than 0.60% | **Lot 1** | 0.00% | 0.00% | 0.00% | 0.00% |
| | **Lot 2** | 0.00% | 0.00% | 0.00% | 0.00% |
| | **Lot 3** | 0.00% | 0.00% | 0.00% | 0.00% |
| Related Compound B<br>No more than 0.90% | **Lot 1** | 0.01% | 0.00% | 0.00% | 0.01% |
| | **Lot 2** | 0.01% | 0.00% | 0.00% | 0.01% |
| | **Lot 3** | 0.01% | 0.01% | 0.00% | 0.01% |
| Any unspecified impurities<br>No more than 0.60% | **Lot 1** | 0.09% | 0.00% | 0.00% | 0.09% |
| | **Lot 2** | 0.12% | 0.00% | 0.00% | 0.12% |
| | **Lot 3** | 0.10% | 0.01% | 0.01% | 0.10% |
| Total impurities<br>No more than 1.50% | **Lot 1** | 0.13% | 0.10% | 0.00% | 0.13% |
| | **Lot 2** | 0.16% | 0.00% | 0.00% | 0.16% |
| | **Lot 3** | 0.13% | 0.02% | 0.03% | 0.13% |

Table 46. Atorvastatin dissolution results

| **Specification** | | **Initial** | **1 month** | **3 months** | **6 months** |
|---|---|---|---|---|---|
| Q = 75.0% in 30 minutes | **Lot 1** | 103.7% | 107.4% | 103.9% | 106.8% |
| | **Lot 2** | 106.2% | 107.1% | 105.6% | 104.5% |
| | **Lot 3** | 106.7% | 106.2% | 103.6% | 105.7% |

Table 47. Fenofibrate dissolution results

| **Specification** | | **Initial** | **1 month** | **3 months** | **6 months** |
|---|---|---|---|---|---|
| Q = 60.0% in 45 minutes | **Lot 1** | 70.2% | 70.4% | 71.4% | 75.9% |
| | **Lot 2** | 69.9% | 72.9% | 70.6% | 71.6% |
| | **Lot 3** | 70.1% | 69.7% | 74.6% | 76.4% |

**Example 14. Stability of pharmaceutical compositions of atorvastatin and fenofibrate 20/200 mg tablets.**

**Accelerated Stability Analytical Results (25°C ± 2°C / 60% RH ± 5% RH)**

Table 48. Product description results

| Specification | | Initial | 1 month | 3 months | 6 months |
|---|---|---|---|---|---|
| White coated biconvex tablet, smooth on both sides | Lot 1 | White coated biconvex tablet, smooth on both sides | White coated biconvex tablet, smooth on both sides | White coated biconvex tablet, smooth on both sides | White coated biconvex tablet, smooth on both sides |
| | Lot 2 | White coated biconvex tablet, smooth on both sides | White coated biconvex tablet, smooth on both sides | White coated biconvex tablet, smooth on both sides | White coated biconvex tablet, smooth on both sides |
| | Lot 3 | White coated biconvex tablet, smooth on both sides | White coated biconvex tablet, smooth on both sides | White coated biconvex tablet, smooth on both sides | White coated biconvex tablet, smooth on both sides |

Table 49. Atorvastatin content results

| Specification | | Initial | 3 months | 6 months |
|---|---|---|---|---|
| 90.0% to 110.0% | **Lot 1** | 100.3% | 98.3% | 99.6% |
| | **Lot 2** | 99.8% | 99.5% | 103.4% |
| | **Lot 3** | 106.3% | 97.0% | 97.0% |

Table 50. Fenofibrate content results

| Specification | | Initial | 3 months | 6 months |
|---|---|---|---|---|
| 90.0% to 110.0% | **Lot 1** | 99.8% | 98.6% | 98.5% |
| | **Lot 2** | 99.9% | 99.3% | 96.9% |
| | **Lot 3** | 101.4% | 95.5% | 95.6% |

Table 51. Organic impurities of atorvastatin results

| Specification | | Initial | 3 months | 6 months |
|---|---|---|---|---|
| Atorvastatin Pyrrolidone Analog<br>No more than 0.90% | **Lot 1** | 0.13% | 0.19% | 0.09% |
| | **Lot 2** | 0.13% | 0.09% | 0.08% |
| | **Lot 3** | 0.13% | 0.06% | 0.08% |
| Related Compound H<br>No more than 1.40% | **Lot 1** | 0.06% | 0.05% | 0.05% |
| | **Lot 2** | 0.06% | 0.05% | 0.05% |
| | **Lot 3** | 0.06% | 0.04% | 0.05% |
| Epoxy Pyrrolooxazin 6-Hydroxy Analog<br>No more than 0.90% | **Lot 1** | 0.00% | 0.20% | 0.16% |
| | **Lot 2** | 0.00% | 0.17% | 0.18% |
| | **Lot 3** | 0.00% | 0.15% | 0.18% |
| Epoxy Pyrrolooxazin 7-Hydroxy Analog<br>No more than 0.90% | **Lot 1** | 0.04% | 0.03% | 0.02% |
| | **Lot 2** | 0.00% | 0.02% | 0.03% |
| | **Lot 3** | 0.00% | 0.01% | 0.02% |
| Epoxy Tetrahydrofuran Analog<br>No more than 1.00% | **Lot 1** | 0.00% | 0.00% | 0.00% |
| | **Lot 2** | 0.00% | 0.00% | 0.00% |
| | **Lot 3** | 0.00% | 0.00% | 0.00% |

(continued)

| Specification | | Initial | 3 months | 6 months |
|---|---|---|---|---|
| Related Compound D when integrated with Epoxy Tetrahydrofuran Analog<br>No more than 0.75% | Lot 1 | 0.02% | 0.03% | 0.02% |
| | Lot 2 | 0.02% | 0.03% | 0.03% |
| | Lot 3 | 0.02% | 0.02% | 0.02% |
| Any other unspecified degradation products<br>Not more than 0.60% | Lot 1 | 0.13% | 0.15% | 0.07% |
| | Lot 2 | 0.14% | 0.07% | 0.06% |
| | Lot 3 | 0.15% | 0.06% | 0.06% |
| Total degradation products<br>No more than 5.00% | Lot 1 | 0.56% | 1.05% | 0.57% |
| | Lot 2 | 0.51% | 0.75% | 0.58% |
| | Lot 3 | 0.51% | 0.71% | 0.57% |

Table 52. Organic impurities of fenofibrate results

| Specification | | Initial | 3 months | 6 months |
|---|---|---|---|---|
| Related Compound A<br>No more than 0.60% | Lot 1 | 0.00% | 0.00% | 0.00% |
| | Lot 2 | 0.00% | 0.00% | 0.00% |
| | Lot 3 | 0.00% | 0.00% | 0.00% |
| Related Compound B<br>No more than 0.90% | Lot 1 | 0.01% | 0.00% | 0.00% |
| | Lot 2 | 0.01% | 0.00% | 0.00% |
| | Lot 3 | 0.01% | 0.00% | 0.00% |
| Any unspecified impurities<br>No more than 0.60% | Lot 1 | 0.09% | 0.01% | 0.01% |
| | Lot 2 | 0.12% | 0.01% | 0.01% |
| | Lot 3 | 0.10% | 0.01% | 0.01% |
| Total impurities<br>No more than 1.50% | Lot 1 | 0.13% | 0.02% | 0.02% |
| | Lot 2 | 0.16% | 0.02% | 0.04% |
| | Lot 3 | 0.13% | 0.01% | 0.04% |

Table 53. Atorvastatin dissolution results

| Specification | | Initial | 3 months | 6 months |
|---|---|---|---|---|
| Q = 75.0% in 30 minutes | Lot 1 | 103.7% | 103.9% | 108.0% |
| | Lot 2 | 106.2% | 98.3% | 107.1% |
| | Lot 3 | 106.7% | 102.5% | 110.3% |

Table 54. Fenofibrate dissolution results

| Specification | | Initial | 3 months | 6 months |
|---|---|---|---|---|
| Q = 60.0% in 45 minutes | Lot 1 | 70.2% | 71.6% | 71.5% |
| | Lot 2 | 69.9% | 72.0% | 71.6% |
| | Lot 3 | 70.1% | 71.6% | 74.3% |

**Example 15. Stability of pharmaceutical compositions of atorvastatin and fenofibrate 20/200 mg tablets.**

**Accelerated Stability Analytical Results (30°C ± 2°C / 75% RH ± 5% RH)**

Table 55. Product description results

| Specification | | Initial | 1 month | 3 months | 6 months |
|---|---|---|---|---|---|
| White coated biconvex tablet, smooth on both sides | Lot 1 | White coated biconvex tablet, smooth on both sides | White coated biconvex tablet, smooth on both sides | White coated biconvex tablet, smooth on both sides | White coated biconvex tablet, smooth on both sides |
| | Lot 2 | White coated biconvex tablet, smooth on both sides | White coated biconvex tablet, smooth on both sides | White coated biconvex tablet, smooth on both sides | White coated biconvex tablet, smooth on both sides |
| | Lot 3 | White coated biconvex tablet, smooth on both sides | White coated biconvex tablet, smooth on both sides | White coated biconvex tablet, smooth on both sides | White coated biconvex tablet, smooth on both sides |

Table 56. Atorvastatin content results

| Specification | | Initial | 3 months | 6 months |
|---|---|---|---|---|
| 90.0% to 110.0% | **Lot 1** | 100.3% | 98.4% | 98.3% |
| | **Lot 2** | 99.8% | 99.6% | 99.6% |
| | **Lot 3** | 106.3% | 103.0% | 106.1% |

Table 57. Fenofibrate content results

| Specification | | Initial | 3 months | 6 months |
|---|---|---|---|---|
| 90.0% to 110.0% | **Lot 1** | 99.8% | 98.6% | 98.6% |
| | **Lot 2** | 99.9% | 98.5% | 99.4% |
| | **Lot 3** | 101.4% | 97.6% | 99.8% |

Table 58. Organic impurities of atorvastatin results

| Specification | | Initial | 3 months | 6 months |
|---|---|---|---|---|
| Atorvastatin Pyrrolidone Analog<br>No more than 0.90% | **Lot 1** | 0.13% | 0.20% | 0.23% |
| | **Lot 2** | 0.13% | 0.19% | 0.23% |
| | **Lot 3** | 0.13% | 0.20% | 0.23% |
| Related Compound H<br>No more than 1.40% | **Lot 1** | 0.06% | 0.05% | 0.05% |
| | **Lot 2** | 0.06% | 0.05% | 0.05% |
| | **Lot 3** | 0.06% | 0.05% | 0.05% |
| Epoxy Pyrrolooxazin 6-Hydroxy Analog | **Lot 1** | 0.00% | 0.22% | 0.28% |
| | **Lot 2** | 0.00% | 0.21% | 0.27% |
| No more than 0.90% | **Lot 3** | 0.00% | 0.20% | 0.28% |

(continued)

| Specification | | Initial | 3 months | 6 months |
|---|---|---|---|---|
| Epoxy Pyrrolooxazin 7-Hydroxy Analog<br>No more than 0.90% | Lot 1 | 0.04% | 0.03% | 0.04% |
| | Lot 2 | 0.00% | 0.00% | 0.04% |
| | Lot 3 | 0.00% | 0.03% | 0.04% |
| Epoxy Tetrahydrofuran Analog<br>No more than 1.00% | Lot 1 | 0.00% | 0.00% | 0.00% |
| | Lot 2 | 0.00% | 0.03% | 0.00% |
| | Lot 3 | 0.00% | 0.00% | 0.00% |
| Related Compound D when integrated with Epoxy Tetrahydrofuran Analog<br>No more than 0.75% | Lot 1 | 0.02% | 0.03% | 0.04% |
| | Lot 2 | 0.02% | 0.03% | 0.04% |
| | Lot 3 | 0.02% | 0.03% | 0.04% |
| Any other unspecified degradation products<br>Not more than 0.60% | Lot 1 | 0.13% | 0.14% | 0.14% |
| | Lot 2 | 0.14% | 0.14% | 0.14% |
| | Lot 3 | 0.15% | 0.14% | 0.15% |
| Total degradation products<br>No more than 5.00% | Lot 1 | 0.56% | 1.11% | 0.95% |
| | Lot 2 | 0.51% | 1.02% | 0.96% |
| | Lot 3 | 0.51% | 1.04% | 0.98% |

Table 59. Organic impurities of fenofibrate results

| Specification | | Initial | 3 months | 6 months |
|---|---|---|---|---|
| Related Compound A<br>No more than 0.60% | Lot 1 | 0.00% | 0.00% | 0.00% |
| | Lot 2 | 0.00% | 0.00% | 0.00% |
| | Lot 3 | 0.00% | 0.00% | 0.00% |
| Related Compound B<br>No more than 0.90% | Lot 1 | 0.01% | 0.00% | 0.00% |
| | Lot 2 | 0.01% | 0.00% | 0.00% |
| | Lot 3 | 0.01% | 0.01% | 0.01% |
| Any unspecified impurities<br>No more than 0.60% | Lot 1 | 0.09% | 0.01% | 0.01% |
| | Lot 2 | 0.12% | 0.00% | 0.01% |
| | Lot 3 | 0.10% | 0.01% | 0.01% |
| Total impurities<br>No more than 1.50% | Lot 1 | 0.13% | 0.00% | 0.04% |
| | Lot 2 | 0.16% | 0.03% | 0.04% |
| | Lot 3 | 0.13% | 0.01% | 0.04% |

Table 60. Atorvastatin dissolution results

| Specification | | Initial | 3 months | 6 months |
|---|---|---|---|---|
| Q = 75.0% in 30 minutes | Lot 1 | 103.7% | 107.4% | 106.3% |
| | Lot 2 | 106.2% | 107.8% | 108.5% |
| | Lot 3 | 106.7% | 110.0% | 107.0% |

Table 61. Fenofibrate dissolution results

| Specification | | Initial | 3 months | 6 months |
|---|---|---|---|---|
| Q = 60.0% in 45 minutes | **Lot 1** | 70.2% | 72.2% | 73.4% |
| | **Lot 2** | 69.9% | 71.8% | 71.4% |
| | **Lot 3** | 70.1% | 71.6% | 71.5% |

**Advantages and applications of the invention**

Solid dosage forms stand out for their high physical, chemical, and biological stability, dosage accuracy, drug release control, and low cost.

Oral liquid forms usually present some advantages such as a higher bioavailability than solid forms, a lower irritant effect on the gastric mucosa, and an easier ingestion. Among the disadvantages are the greater likelihood of contamination and the possible instability of drugs in dissolution. A sign of instability of the solutions is that they may crystallize since the medium in which they are found may favor the change to its base state making it less soluble and which in turn decreases the bioavailability of the drug.

This invention describes an optimized method that permits the combined manufacture of the active principles atorvastatin and fenofibrate at a dosage of 20/200 mg and 20/160 mg respectively, in a single solid, stable, immediate release dosage form. Atorvastatin is preferably in its calcium trihydrate form. Thus, this invention conducts a process of granulation activated by heating with fenofibrate and incorporates atorvastatin to carry out the manufacture without the incorporation of water and avoiding conditions that could affect the stability of this drug.

Thus, this invention solves a set of important technological challenges due to the physicochemical properties and the difference in dosage to ensure obtaining a stable product that would otherwise be impossible to obtain or formulate.

The composition of this invention is also intended for the treatment and control of the selected group of conditions of hyperlipidemia, hyperlipoproteinemia, hypercholesterolemia, as well as the prevention and control of cardiovascular diseases such as hypertension, angina pectoris, heart attacks, aneurysms, regulation of the blood concentration of LDL, HDL, and triglycerides, among others.

References

Back HM, Song B, Pradhan S, ChaeJW, Han N, Kang W, Chang MJ, Zheng J, Kwon KI, Karlsson MO, Yun HY. A mechanism-based pharmacokinetic model of fenofibrate for explaining increased drug absorption after food consumption. BMC Pharmacology and Toxicology (2018) 19:4, pp 1-10.

Chou YC, Wang YK, Charng MJ, Ueng YF. Determination of serum atorvastatin concentrations in lipid-controlling patients with and without myalgia syndrome. Journal of Food and Drug Analysis, Volume 21, Issue 2, June 2013, Pages 147-153.

Food and Drug Administration (FDA), Center for Drug Evaluation and Research (CDER). Clinical Drug Interaction Studies -Cytochrome P450 Enzyme- and Transporter-Mediated Drug Interactions Guidance for Industry. January 2020 Clinical Pharmacology.

Lins RL, Matthys KE, Verpooten GA, Peeters PC, Drafinra M, Stolear JC, Lameire NH. Pharmacokinetics of Atorvastatin and Its Metabolites After Single and Multiple Dosing in Hypercholesterolaemic Haemodialysis Patients. Nephrol Dial Transplant. 2003 May;18(5):967-76.

LIPITOR® (atorvastatin calcium tablets), Product monograph 10 mg, 20 mg, 40 mg, and 80 mg atorvastatin. Lipid metabolism regulator. Submission Control No: 237270. ®Pfizer Ireland Pharmaceuticals Upjohn Canada ULC, Licensee © Upjohn Canada ULC, 2020. Date of Revision: April 30, 2020; page 27.

Moffat AC, Osselton MD, Widdop B. Clarke's Analysis of Drugs and Poisons in pharmaceuticals, body fluids and postmortem material. Fourth edition, Pharmaceutical Press 2011: 42

Reddy P, Ellington D, Zhu Y, Zdrojewski I, Parent SJ, Harmatz JS, Derendorf H, Greenblatt DJ, Browne J. Serum concentrations and clinical effects of atorvastatin in patients taking grapefruit juice daily. British Journal of Clinical Pharmacology © 2011, 72:3 / 434-441.

Wong B.A. Focus on Statin Research. Nova Biomedical Books, New York, 2006: 112-130.

Yamazaki M, Li B, Louie SW, Pudvah NT, Stocco R, Wong W, Abramovitz M, Demartis A, Laufer R, Hochman JH, Prueksaritanont T, Lin JH. Effects of Fibrates on Human Organic Anion-Transporting Polypeptide 1B1-, Multidrug Resistance Protein 2- And P-glycoprotein-mediated Transport. Xenobiotica. 2005 Jul;35(7):737-53.

## Claims

1. A pharmaceutical composition **characterized by** comprising: (a) atorvastatin, or an equivalent amount of a salt thereof, in a pharmaceutically acceptable amount in the range between 20 $\pm$ 0.7 mg and (b) micronized fenofibrate, or its micronized salt, in a pharmaceutically acceptable amount in the range between 160 mg and 200 mg, and (c) a pharmaceutically acceptable amount of pharmaceutically acceptable excipients and/or pharmaceutical acceptable vehicles; and wherein said composition is further in a solid, stable, immediate release form.

2. The pharmaceutical composition according to claim 1, **characterized in that** the atorvastatin is preferably in its calcium salt trihydrate form in an amount of 21.65 mg equivalent to 20 mg of atorvastatin.

3. The pharmaceutical composition according to claim 1, **characterized in that** the micronized fenofibrate has a particle size distribution of less than 30 $\mu$m (100%).

4. The pharmaceutical composition according to claim 3, **characterized in that** the particle size distribution of the micronized fenofibrate is preferably equal to or less than 0.95 $\mu$m (d10).

5. The pharmaceutical composition according to claim 3, **characterized in that** the particle size distribution of the micronized fenofibrate is preferably equal to or less than 5.35 $\mu$m (d50).

6. The pharmaceutical composition according to claim 3, **characterized in that** the particle size distribution of the micronized fenofibrate is preferably equal to or less than 11.28 $\mu$m (d90).

7. The pharmaceutical composition according to claims 1 to 6, **characterized in that** the pharmaceutically acceptable excipients and/or vehicles comprise binders, diluents, disintegrants, pH modulators, surfactants, lubricants, solvents, and coatings.

8. The pharmaceutical composition according to claim 7, **characterized in that** the binder is preferably poloxamer 188 in a pharmaceutically acceptable amount in the range of 5 to 10%.

9. The pharmaceutical composition according to claim 7, **characterized in that** the diluent is preferably lactose monohydrate in a pharmaceutically acceptable amount in the range of 5 to 90%.

10. The pharmaceutical composition according to claim 7, **characterized in that** the diluent is preferably magnesium aluminum silicate in a pharmaceutically acceptable amount in the range of 5 to 90%.

11. The pharmaceutical composition according to claim 7, **characterized in that** the disintegrant is preferably starch sodium glycolate in a pharmaceutically acceptable amount in the range of 2 to 8%.

12. The pharmaceutical composition according to claim 7, **characterized in that** the pH modulator is preferably magnesium oxide in a pharmaceutically acceptable amount in the range of 0.5 to 5%.

13. The pharmaceutical composition according to claim 7, **characterized in that** the surfactant is preferably sodium lauryl sulfate in a pharmaceutically acceptable amount in the range of 1 to 2.5%.

14. The pharmaceutical composition according to claim 7, **characterized in that** the lubricant is preferably magnesium stearate in a pharmaceutically acceptable amount in the range of 0.25 to 5%.

15. The pharmaceutical composition according to claim 7, **characterized in that** the coating is a moisture barrier coating.

16. The pharmaceutical composition according to claim 15, **characterized in that** the moisture barrier coating is preferably AMB II Opadry in a pharmaceutically acceptable amount in the range of 0.5 to 6%.

17. The pharmaceutical composition according to claim 7, **characterized in that** the solvent is preferably water in a pharmaceutically acceptable amount.

18. The pharmaceutical composition according to any of claims 1-17, **characterized in that** the solid pharmaceutical form is selected from the group comprising a tablet, caplet, granules, pills.

**19.** The pharmaceutical composition in accordance with claim 18, **characterized in that** it is preferably in the form of a biconvex tablet or coated tablet.

**20.** A process of manufacturing the pharmaceutical composition according to claims 1-19, **characterized in that** it comprises:

- a stage of granulation activated by heating with fenofibrate, preferably micronized fenofibrate.
- a stage of incorporation of the atorvastatin.
- a compression stage.

wherein the process is carried out without the incorporation of water avoiding conditions affecting the stability of the atorvastatin.

**21.** The process according to claim 20, **characterized in that** the heating activated granulation stage comprises:

a) Adding 70% magnesium oxide to the micronized fenofibrate and manually mixing to remove static from the powder.
b) Sieving the above mixture by mesh (0.64 mm or 0.025 in.), together with poloxamer 188, 20% lactose monohydrate DCL-11, and 80% magnesium aluminum silicate.
c) Loading the powder in a granulating equipment with heating system and bring to a temperature of 47°C with constant movement.
d) When the temperature is reached, stop the heating and the granulate obtained is left to cool to 25-30°C.
e) Performing a particle size reduction operation by mesh (1.27 mm or 0.050 in.) to the granules obtained in step c).

**22.** The process according to claim 20, **characterized in that** the step of incorporating the atorvastatin comprises:

f) Adding the atorvastatin and 20% magnesium aluminum silicate in a diffusion mixer, mixing for 3 minutes.
g) Sieving the sodium starch glycolate, 30% magnesium oxide, and 80% lactose monohydrate DCL-11 through a sieve (1.27 mm or 0.050 in.).
h) Adding to the mixer of step f), the sieved from step e), the powder from step g), and mixing for 5 min.
i) Sieving through a mesh (1.27 mm or 0.050 in.) the sodium lauryl sulfate and magnesium stearate.
j) Adding the powder of step 9 to the mixer from step 8 and mixed for 3 min.

**23.** The process according to claim 20, **characterized in that** the compressing step comprises:

k) Compressing the final powder mixture into 600 $\pm$ 30 mg nuclei, with a hardness of 6.0-13.0 Kp and disintegration time of less than 9 min.
l) Coating the cores with 3.23% of Opadry AMB II in weight gain.

**24.** Use of the pharmaceutical composition of any one of claims 1-19 for the manufacture of a drug product useful in the treatment of hyperlipidemias and the prevention of cardiovascular diseases.

**25.** The use, according to claim 24, wherein the hyperlipidemias and cardiovascular diseases are selected from: hyperlipidemia, hyperlipoproteinemia, hypercholesterolemia, hypertension, angina pectoris, heart attacks, aneurysms, regulation of LDL, HDL, and triglyceride blood concentration.

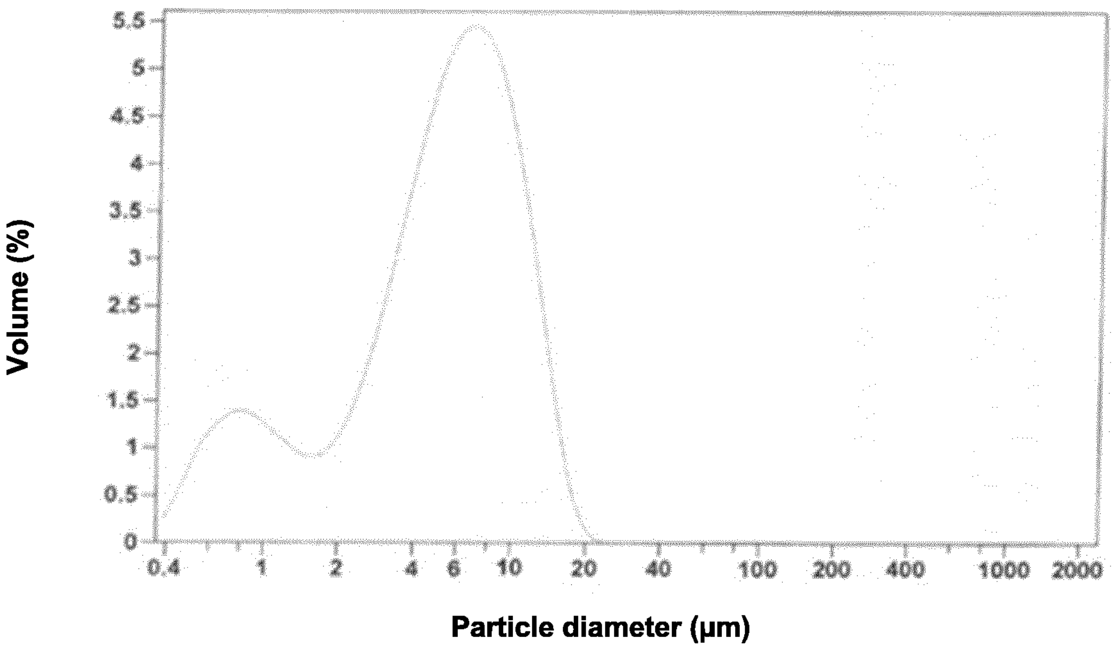


Figure 1

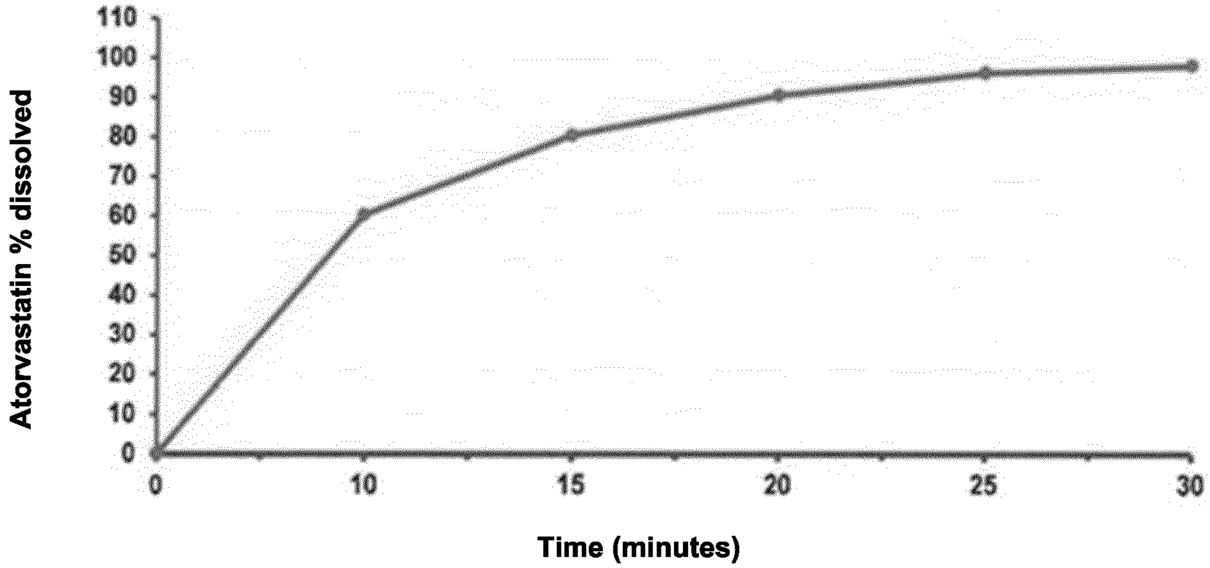


Figure 2

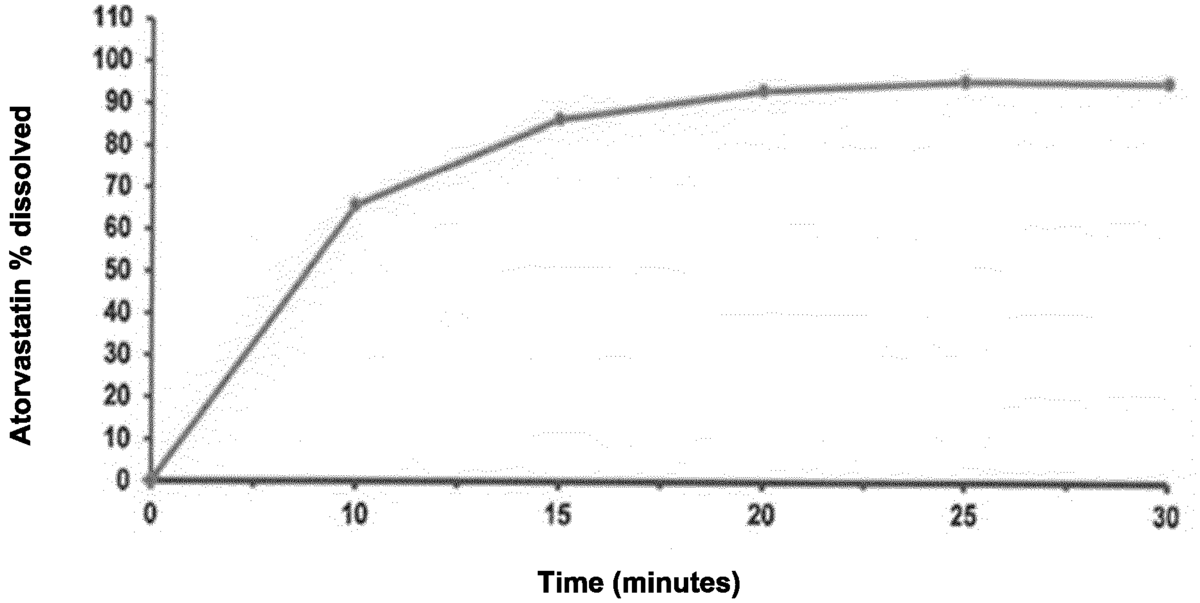


Figure 3

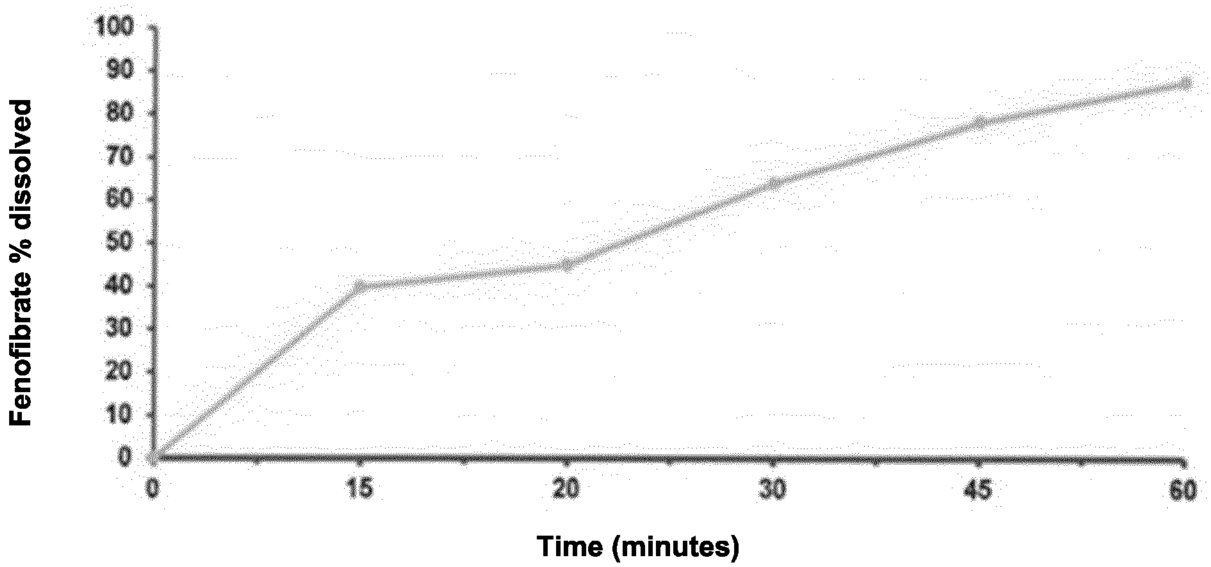


Figure 4

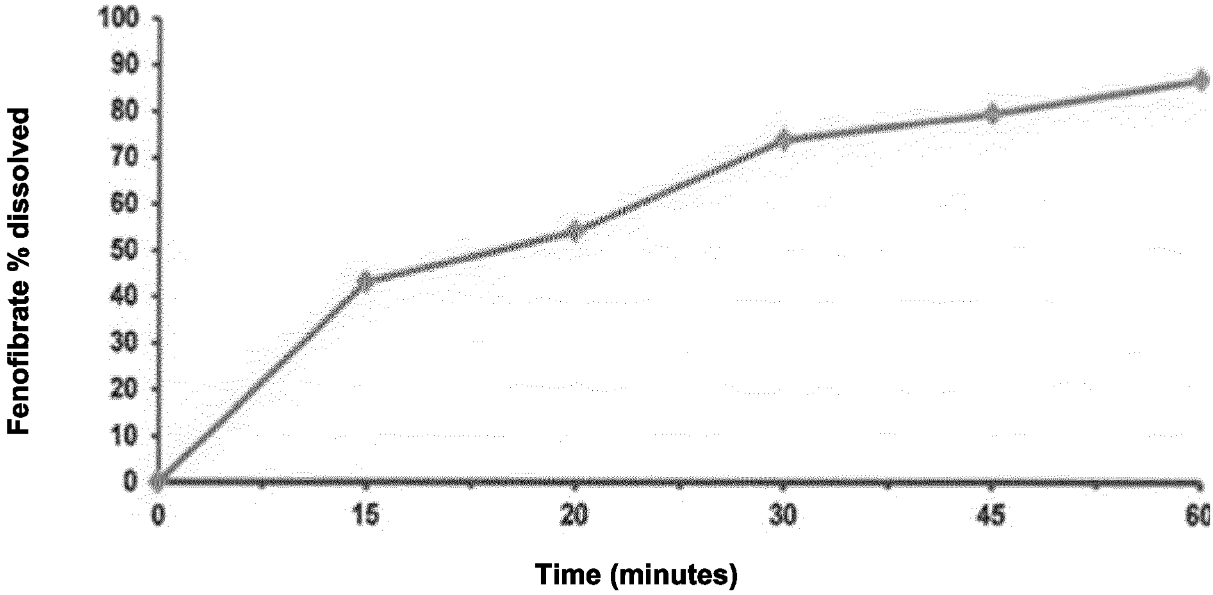


Figure 5

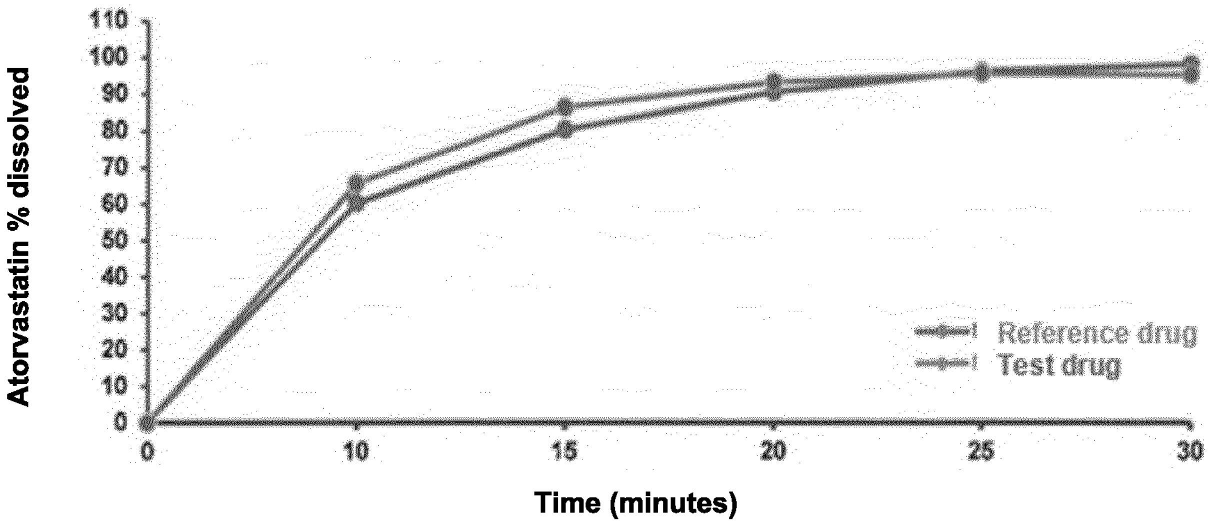


Figure 6

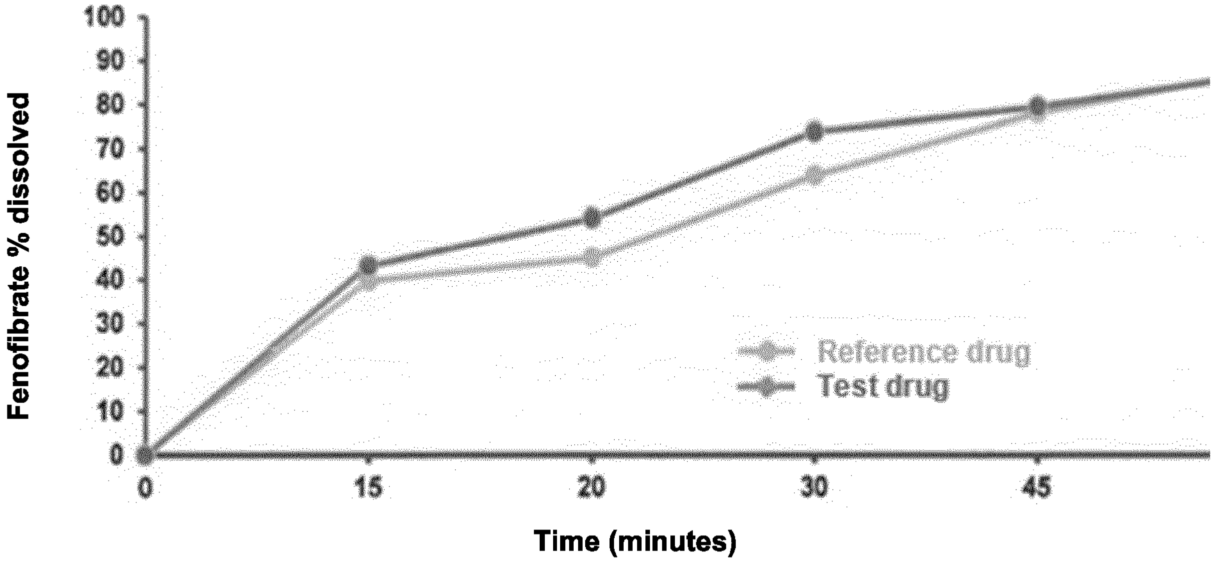


Figure 7

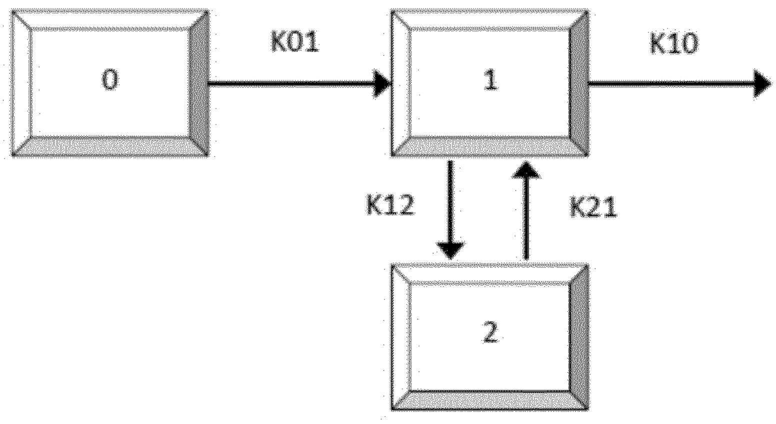


Figure 8

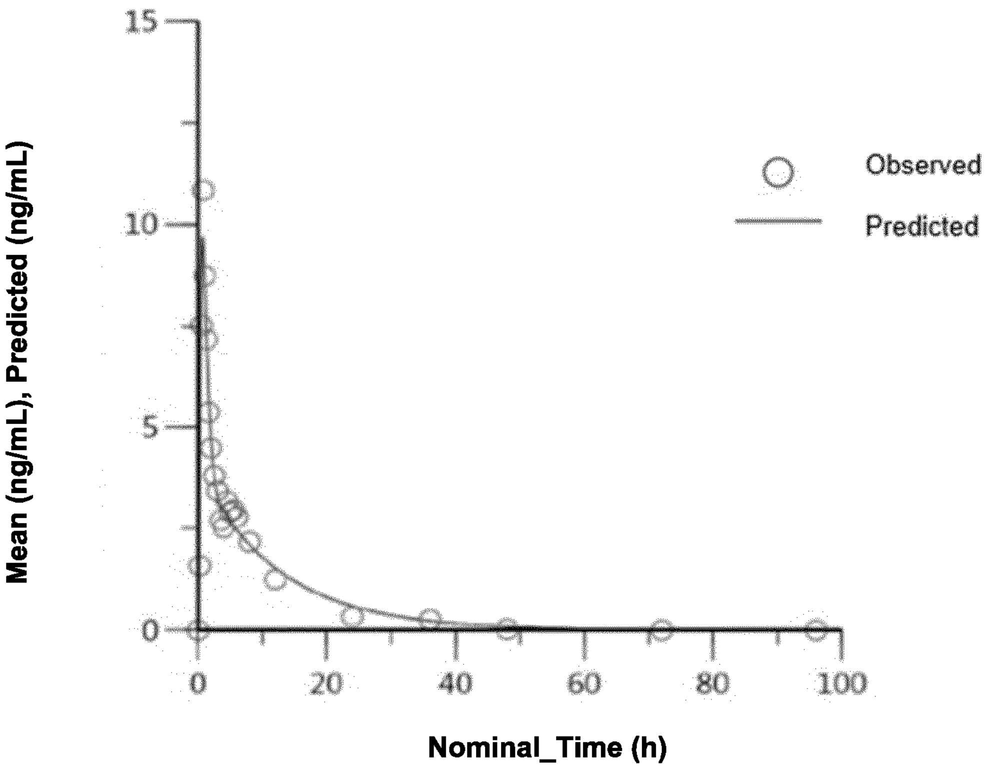


Figure 9

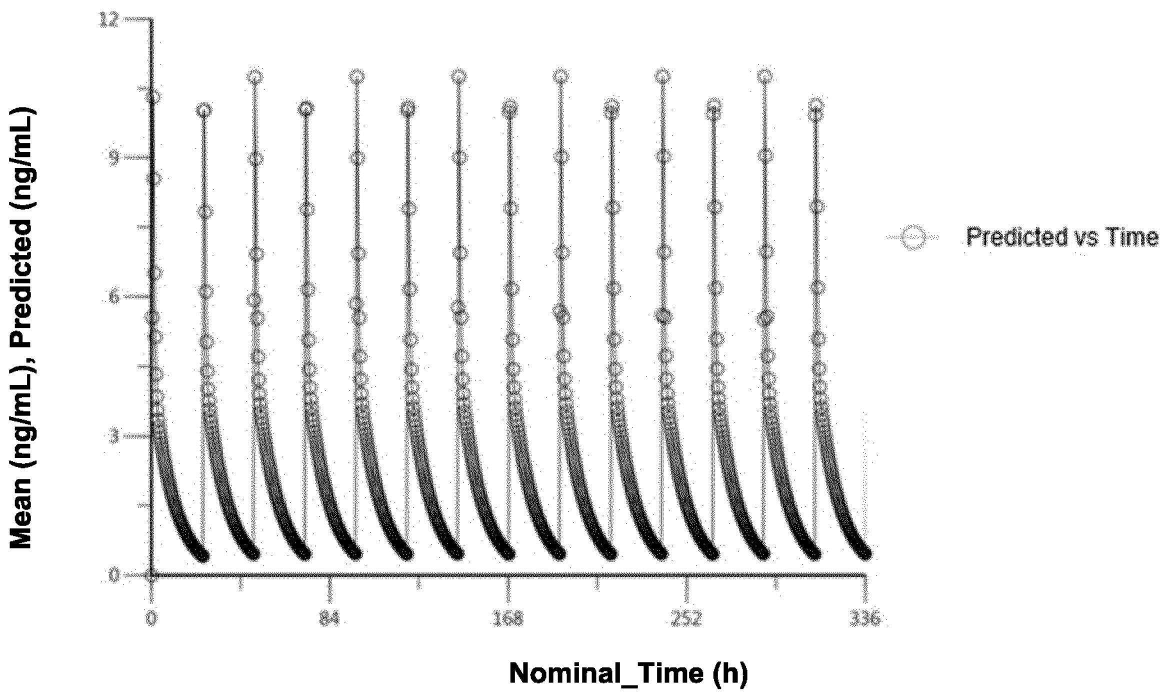


Figure 10

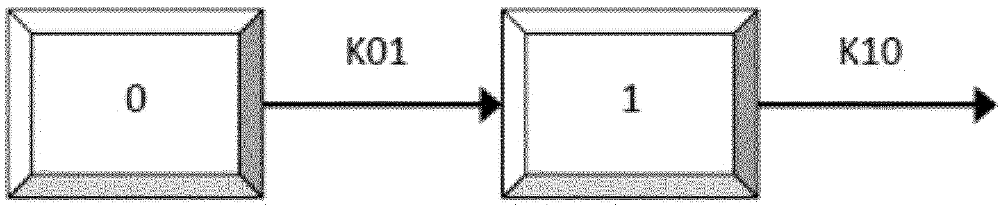


Figure 11

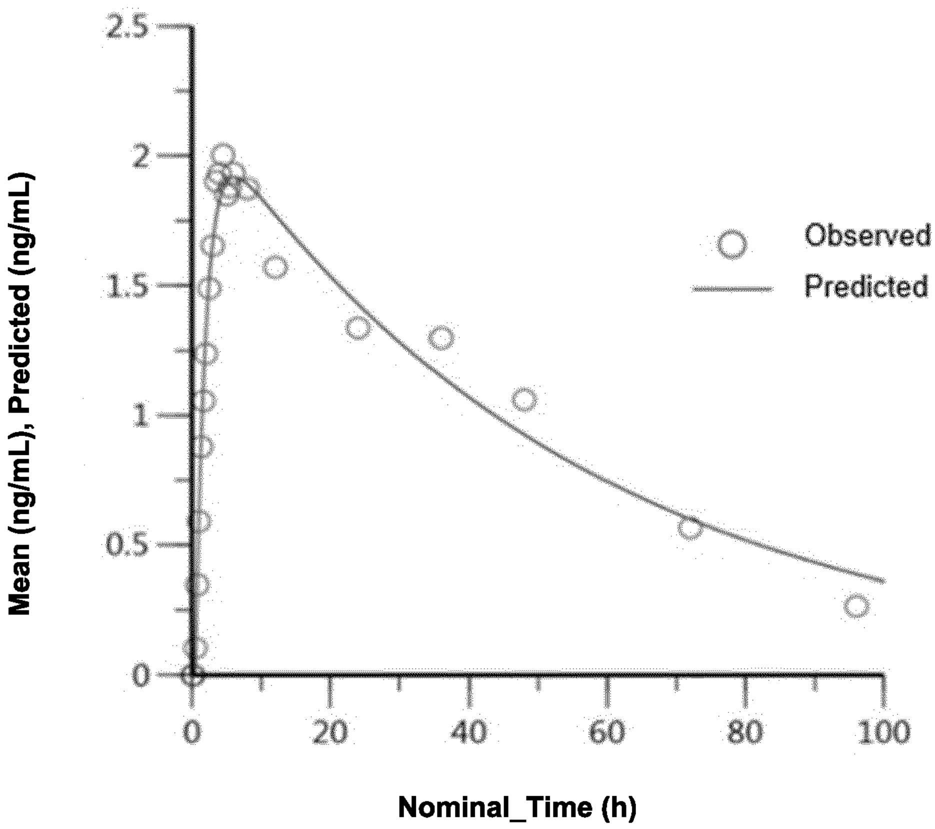


Figure 12

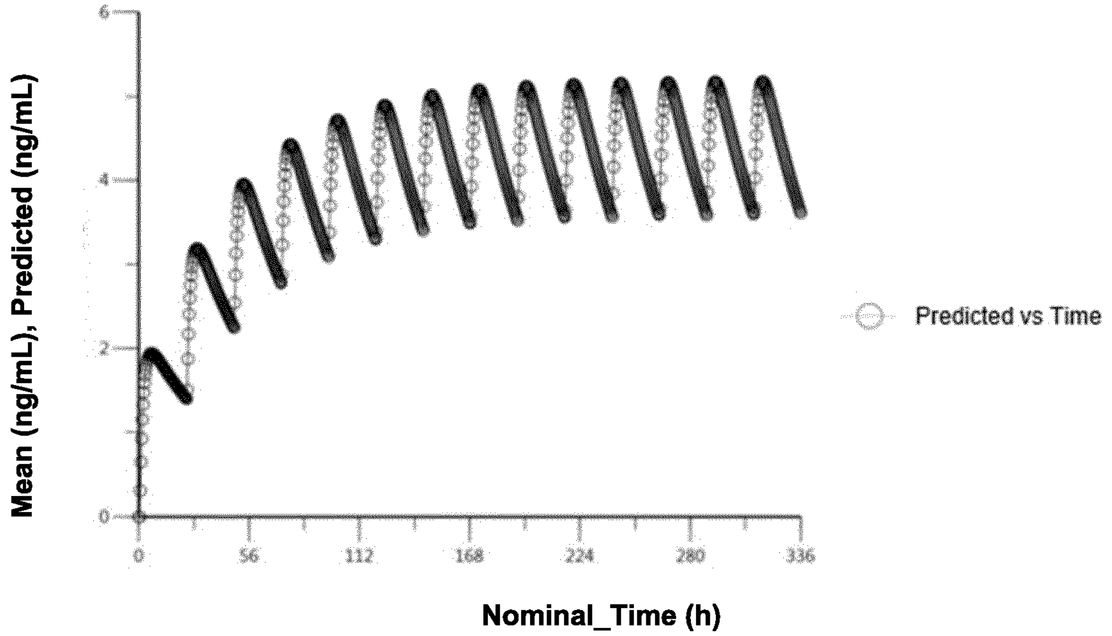


Figure 13

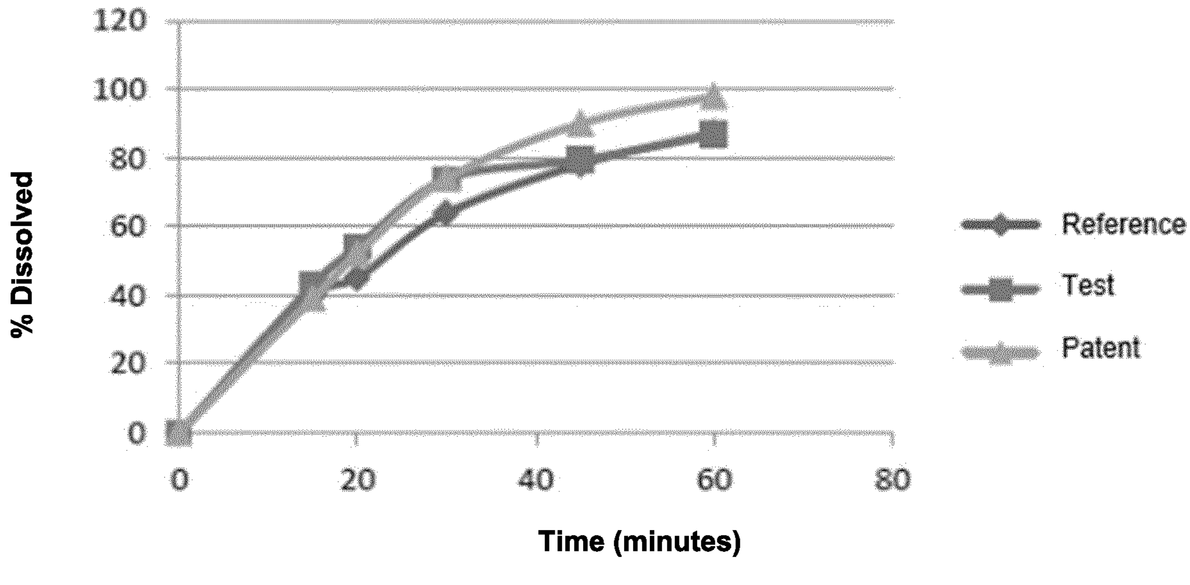


Figure 14

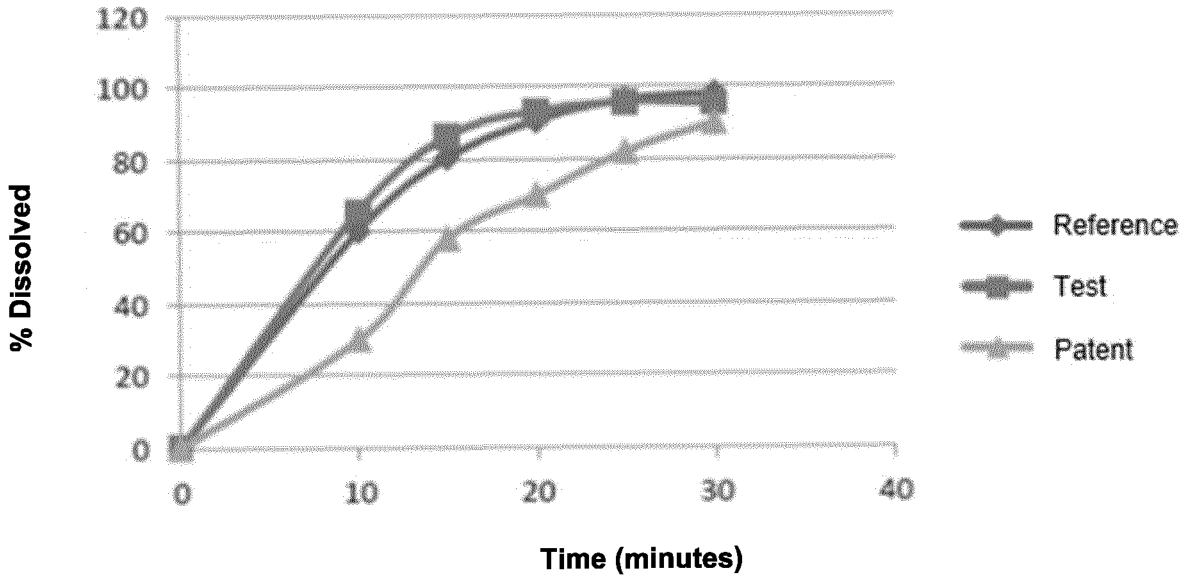
120
100
80
60
40
20
0
% Dissolved
0
10
20
30
40
Time (minutes)
Reference
Test
Patent

Figure 15

| INTERNATIONAL SEARCH REPORT | International application No. PCT/MX2020/050034 |
|---|---|

A. CLASSIFICATION OF SUBJECT MATTER

**See extra sheet**

According to International Patent Classification (IPC) or to both national classification and IPC

B. FIELDS SEARCHED

Minimum documentation searched (classification system followed by classification symbols)
A61P

Documentation searched other than minimum documentation to the extent that such documents are included in the fields searched

Electronic data base consulted during the international search (name of data base and, where practicable, search terms used)

INVENES, PATENW, BIOSIS, EMBASE, MEDLINE, NPL, XPESP

C. DOCUMENTS CONSIDERED TO BE RELEVANT

| Category* | Citation of document, with indication, where appropriate, of the relevant passages | Relevant to claim No. |
|---|---|---|
| X | WO 2014195900 A2 (ALPARIS S A DE C V) 11/12/2014, page 6, lines 19-27, page 7, lines 14-17, page 31, page 35, lines 22-25, examples 3 and 4, claims 7-18, 22. | 1-25 |
| X | WO 2006037344 A1 (LIFECYCLE PHARMA AS ET AL.) 13/04/2006, examples 1-9, paragraph 178, claims 44-46. | 1-25 |
| X | US 2007009603 A1 (HOLM PER ET AL.) 11/01/2007, paragraphs 271- 277, examples 1-4, claims 59 to 65 | 1-25 |

☒ Further documents are listed in the continuation of Box C. ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance. | |
| "E" earlier document but published on or after the international filing date | |
| "L" document which may throw doubts on priority claim(s) or which is cited to establish the publication date of another citation or other special reason (as specified) | "X" document of particular relevance; the claimed invention cannot be considered novel or cannot be considered to involve an inventive step when the document is taken alone |
| "O" document referring to an oral disclosure use, exhibition, or other means. | "Y" document of particular relevance; the claimed invention cannot be considered to involve an inventive step when the document is combined with one or more other documents , such combination being obvious to a person skilled in the art |
| "P" document published prior to the international filing date but later than the priority date claimed | |
| | "&" document member of the same patent family |

| | |
|---|---|
| Date of the actual completion of the international search<br>20/06/2021 | Date of mailing of the international search report<br>**(23/06/2021)** |
| Name and mailing address of the ISA/<br><br>OFICINA ESPAÑOLA DE PATENTES Y MARCAS<br>Paseo de la Castellana, 75 - 28071 Madrid (España)<br>Facsimile No.: 91 349 53 04 | Authorized officer<br>H. Aylagas Cancio<br><br>Telephone No. 91 3498563 |

Form PCT/ISA/210 (second sheet) (January 2015)

## INTERNATIONAL SEARCH REPORT

| International application No. |
|---|
| PCT/MX2020/050034 |

C (continuation). DOCUMENTS CONSIDERED TO BE RELEVANT

| Category * | Citation of documents, with indication, where appropriate, of the relevant passages | Relevant to claim No. |
|---|---|---|
| A | DAVIDSON et al.. Efficacy and tolerability of atorvastatin/fenofibrate fixed-dose combination tablet compared with atorvastatin and fenofibrate monotherapies in patients with dyslipidemia: A 12-week, multicenter, double-blind, randomized, parallel-group study. Clinical Therapeutics, 2009, vol 31, nº.12. pages 2824-2838 the whole document. | 1-25 |
| A | SOBHITA RANI P et al.. Enhancement of bioavailability of Atorvastatin and Fenofibrate combination using solid dispersion technique. Pharmaceutical Sciences and Research ind. 0104, vol.5, nº 9, pages 3713-3725 the whole document. | 1-25 |
| A | DHIWARE A D et al.. A simple and sensitive RP-HPLC method for simultaneous estimation of Atorvastatin Calcium, Ezetimibe and Fenofibrate in combined tablet dosage form. Research Journal of Pharmacy and Technology 2013 Research Journal of Pharmacy and Technology Ind., 30/11/2012, Vol. 6, Nº 10, pages 1085 - 1088, ISSN 0974-360X (print) ISSN 0974-3618 (electronic) the whole document. | 1-25 |

Form PCT/ISA/210 (continuation of second sheet) (January 2015)

# INTERNATIONAL SEARCH REPORT

International application No.

PCT/MX2020/050034

## CLASSIFICATION OF SUBJECT MATTER

***A61K31/40*** (2006.01)
***A61K31/216*** (2006.01)
***A61P3/06*** (2006.01)

Form PCT/ISA/210 (extra sheet) (January 2015)

## INTERNATIONAL SEARCH REPORT

Information on patent family members

International application No.
PCT/MX2020/050034

| Patent document cited in the search report | Publication date | Patent family member(s) | Publication date |
|---|---|---|---|
| WO2006037344 A1 | 13.04.2006 | US2007014846 A1 | 18.01.2007 |
| | | US2007026062 A1 | 01.02.2007 |
| | | US9173847 B2 | 03.11.2015 |
| | | EP1804769 A1 | 11.07.2007 |
| | | EP1804768 A1 | 11.07.2007 |
| | | WO2006037347 A1 | 13.04.2006 |
| | | CA2582405 A1 | 13.04.2006 |
| | | CA2582403 A1 | 13.04.2006 |
| WO2014195900 A2 | 11.12.2014 | MX2013006332 A | 19.12.2014 |
| US2007009603 A1 | 11.01.2007 | US2013303612 A1 | 14.11.2013 |
| | | KR20130103818 A | 24.09.2013 |
| | | US2012195965 A1 | 02.08.2012 |
| | | US8481078 B2 | 09.07.2013 |
| | | US2010323008 A1 | 23.12.2010 |
| | | US8124125 B2 | 28.02.2012 |
| | | HK1096034 A1 | 25.05.2007 |
| | | AU2009201881 A1 | 04.06.2009 |
| | | US2005096391 A1 | 05.05.2005 |
| | | US2006105050 A1 | 18.05.2006 |
| | | US2005096390 A1 | 05.05.2005 |
| | | US2006110444 A1 | 25.05.2006 |
| | | US7658944 B2 | 09.02.2010 |
| | | US2006068015 A1 | 30.03.2006 |
| | | US2007014846 A1 | 18.01.2007 |
| | | US2007026062 A1 | 01.02.2007 |
| | | US9173847 B2 | 03.11.2015 |
| | | RU2006115596 A | 27.11.2007 |
| | | RU2343905 C2 | 20.01.2009 |
| | | MXPA06003813 A | 14.06.2006 |
| | | KR20060085686 A | 27.07.2006 |
| | | KR20060085682 A | 27.07.2006 |
| | | JP2007508249 A | 05.04.2007 |
| | | JP2007508248 A | 05.04.2007 |
| | | JP5069001B B2 | 07.11.2012 |
| | | EP1680086 A2 | 19.07.2006 |
| | | EP1680091 A1 | 19.07.2006 |
| | | EP1680091 B1 | 31.05.2017 |
| | | WO2005034908 A2 | 21.04.2005 |
| | | WO2005034908 A3 | 11.08.2005 |
| | | WO2005034920 A1 | 21.04.2005 |
| | | CN1874758 A | 06.12.2006 |
| | | CN1867321 A | 22.11.2006 |
| | | CN100551363C C | 21.10.2009 |
| | | CA2541382 A1 | 21.04.2005 |
| | | CA2540984 A1 | 21.04.2005 |
| | | CA2540984 C | 08.02.2011 |
| | | BRPI0415121 A | 28.11.2006 |
| | | AU2004279661 A1 | 21.04.2005 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5273995 A
- MX 178535
- MX 214428
- MX 210754
- MX 238174
- MX 263277
- MX 238946
- MX 260435
- MX 2013015272 A
- FR 2157853, FOURNIER GMBH LAB
- MX 252667, ALKERMES PHARMA
- MX 333517, ABBOTT LABORATORIES
- MX 270015, SARL GALENIX INNOVATIONS
- MX 261110
- WO 2014195900 A2
- WO 2006037344 A1
- CN 102188419
- US 20110165239 A1
- MX 2013006332 A
- MX 2006003813 A
- MX 2010014200 A


### Non-patent literature cited in the description


- Use (International Union of Pure and Applied Chemistry. Wiley-VCH, 16 May 2011
- Remington Pharmaceuticals. Editorial Médica Panamericana
- **LACHMAN L ; LIEBERMAN HA ; KANIG JL.** The theory and practice of industrial pharmacy. Lea-Febiger, 1986, 333
- United States Pharmacopeia USP. 2018
- **BACK HM ; SONG B ; PRADHAN S ; CHAEJW ; HAN N ; KANG W ; CHANG MJ ; ZHENG J ; KWON KI ; KARLSSON MO.** A mechanism-based pharmacokinetic model of fenofibrate for explaining increased drug absorption after food consumption. *BMC Pharmacology and Toxicology,* 2018, vol. 19 (4), 1-10
- **CHOU YC ; WANG YK ; CHARNG MJ ; UENG YF.** Determination of serum atorvastatin concentrations in lipid-controlling patients with and without myalgia syndrome. *Journal of Food and Drug Analysis,* June 2013, vol. 21 (2), 147-153
- Clinical Drug Interaction Studies -Cytochrome P450 Enzyme- and Transporter-Mediated Drug Interactions Guidance for Industry. *Clinical Pharmacology,* January 2020
- **LINS RL ; MATTHYS KE ; VERPOOTEN GA ; PEETERS PC ; DRAFINRA M ; STOLEAR JC ; LAMEIRE NH.** Pharmacokinetics of Atorvastatin and Its Metabolites After Single and Multiple Dosing in Hypercholesterolaemic Haemodialysis Patients. *Nephrol Dial Transplant,* May 2003, vol. 18 (5), 967-76
- LIPITOR® (atorvastatin calcium tablets), Product monograph 10 mg, 20 mg, 40 mg, and 80 mg atorvastatin. Lipid metabolism regulator. Pfizer Ireland Pharmaceuticals, 30 April 2020, 27
- **MOFFAT AC ; OSSELTON MD ; WIDDOP B. CLARKE'S.** Analysis of Drugs and Poisons in pharmaceuticals, body fluids and postmortem material. Pharmaceutical Press, 2011, 42
- **REDDY P ; ELLINGTON D ; ZHU Y ; ZDROJEWSKI I ; PARENT SJ ; HARMATZ JS ; DERENDORF H ; GREENBLATT DJ ; BROWNE J.** Serum concentrations and clinical effects of atorvastatin in patients taking grapefruit juice daily. *British Journal of Clinical Pharmacology,* 2011, vol. 72 (3), 434-441
- **WONG B.A.** Focus on Statin Research. Nova Biomedical Books, 2006, 112-130
- **YAMAZAKI M ; LI B ; LOUIE SW ; PUDVAH NT ; STOCCO R ; WONG W ; ABRAMOVITZ M ; DEMARTIS A ; LAUFER R ; HOCHMAN JH.** Effects of Fibrates on Human Organic Anion-Transporting Polypeptide 1B1-, Multidrug Resistance Protein 2- And P-glycoprotein-mediated Transport. *Xenobiotica,* July 2005, vol. 35 (7), 737-53